# EUROPEAN PATENT APPLICATION

(11) **EP 4 015 641 A1**
(43) Date of publication of application: **22.06.2022**
(21) Application number: 20214602.3
(22) Date of filing: 16.12.2020
(51) Int. Cl.: C12P 19/02, C12N 1/14, C12N 9/24, C12N 15/63, C12P 21/00

(54) **PROCESS FOR THE PRODUCTION OF A FILAMENTOUS FUNGUS WHOLE BROTH ENZYME COMPOSITION WITH LOW BIOMASS FORMATION AND HIGH PROTEIN YIELD**

(71) Applicant: Clariant Produkte (Deutschland) GmbH, 65929 Frankfurt am Main (DE)
(72) Inventor: GAMAUF, Christian, 81241 München (DE); CLAREN, Jörg, 82131 Stockdorf (DE)
(74) Representative: Graser, Konstanze

(57) **Abstract**

The present invention relates to a process for the production of a filamentous fungus whole broth enzyme composition with low biomass formation and high protein yield, a genetically modified filamentous fungus cell for production of the whole broth enzyme composition, the use of such a genetically modified filamentous fungus cell for the production of the filamentous fungus whole broth enzyme composition with low biomass formation and high protein yield and a filamentous fungus whole broth enzyme composition produced by such a method.

## Description

The present invention relates to a process for the production of a filamentous fungus whole broth enzyme composition with low biomass formation and high protein yield, a genetically modified filamentous fungus cell for production of the whole broth enzyme composition, the use of such a genetically modified filamentous fungus cell for the production of the filamentous fungus whole broth enzyme composition with low biomass formation and high protein yield and a filamentous fungus whole broth enzyme composition produced by such a method.

Hydrolysate from lignocellulose-containing (or lignocellulosic) biomass is coming more and more into focus as a valuable substrate for the production of various substances such as enzymes, lactic acid, fatty acids, iso-butanol, butanediol, succinic acid, itaconic acid but also ethanol. Ethanol originating from such a process is usually referred to as "bioethanol" or "biofuel". Production of the desired substances is often carried out by fermentation processes involving yeasts, bacteria or fungi capable of producing the desired end product.

One major drawback of such processes is the lignocellulosic biomass substrate itself. All biomasses usually referred to as "lignocellulosic substrate" contain a considerable amount of lignin (up to 40 wt.-%), cellulose (up to 55 wt.-%) and hemicelluloses (up to 55 wt.-%). Due to its origin (wood or weed plant-derived biomass) also the structure of the cell walls is significantly different from those of most other plant species which will influence hydrolysis of the substrate. Hydrolysis can be achieved by application of chemicals such as acids but is usually carried out by digestion of enzymes which will not contaminate the hydrolysate with e.g. salts resulting from chemical treatments.

The necessary hydrolytic breakdown of those polymers involves the use of several cellobiohydrolases, endoglucanases, β-glucosidases and oxidoreductases. To avoid costly supplementation of the single specific enzymes such hydrolytic breakdown is usually carried out by applying a so called "whole broth enzyme composition" produced by a microorganism, such as a filamentous fungus, capable of producing all enzymes necessary for hydrolysis of the substrate. The resulting glucose and cello-oligosaccharides can then easily be fermented to the desired end product - such as ethanol when using ordinary baker's yeast.

To attain economic feasibility, a high yield of the desired end product but also high yield of the produced whole broth enzyme composition is a necessity. This applies in particular when the end product is bioethanol which has to compete with ordinary and cheap mineral-oil derived fuel products on the market. Producing monomeric sugars from cellulose and hemicellulose at high yields is far more difficult than deriving sugars from sugar- or starch-containing crops, e.g. sugarcane or maize (Van Dyck and Pletschke, 2012). Therefore, although the cost of lignocellulosic biomass is far lower than that of sugar and starch crops, the cost of obtaining sugars from such materials for fermentation into e.g. bioethanol has often been considered to be too high to be industrially feasible. For this reason, it is crucial to solve the problems involved in the conversion of lignocellulosic biomass to hydrolysate.

One problem is the high viscosity of the fermentation broth of the fungus, especially of a filamentous fungus, which is needed for production of the whole broth enzyme composition. In order to obtain a high yield of enzymes, a strong growth of the fungus is desired, however, a strong growth comes with a high content of fungus biomass within the fermentation broth. Fungi, which are known to consist of i.a. hyphae are known within the art as rendering any fermentation substrate into a high-viscous composition. This effect is significantly more distinct when a filamentous fungus is used which exhibits a sponge-like, slimy appearance.

High viscosity causes many problems, as the fungus needs constant oxygen supply by aeration during growth. In addition, cooling of the fermenter, especially in industrial-scale production is required. Both can only be guaranteed by constant stirring - on the one hand to distribute the air bubbles homogenously within the broth, and on the other hand to facilitate constant heat-exchange with the cooling devices. The higher the viscosity of the broth the more energy needs to be spent to realize effective stirring within the reactor. Further, more air as to be pressed into the reactor causing also higher energy consumption within the compressor and sterile-filter unit. Thus, both CAPEX and OPEX increase with increasing viscosity of the fermentation broth. An alternative measure - less cell mass production - is also not attractive for commercial production as this would always be accompanied by a lower yield of whole broth enzyme production.

As lignocellulosic biomass is a substrate which already provides several challenges any further cost increase has to be avoided when applying such processes to commercial scale production.

The inventors of the present invention have therefore set themselves the task to develop a process for the production of filamentous fungus whole broth enzyme composition with low biomass formation while maintaining a high yield of enzymes within the broth.

The task has been solved by a process for production of a whole broth enzyme composition, comprising the following steps:
(a) providing a fermentation medium, originating from hydrolysis of lignocellulosic biomass, with a glucose content of from 5 to 450 g/L, a xylose content of from 2 to 300 g/L, a density of from 1 to 2 kg/L and a dry matter content of from 10 to 75 wt.-%;
(b) addition of at least one filamentous fungus cell wherein the A at position 149 of SEQ ID NO:1 has been changed to a G;
(c) mixing of the fermentation medium and the at least one filamentous fungus cell for a time period of from 1 minute to 10 days at a temperature of from 20 to 35 °C;
(d) obtaining a whole broth enzyme composition.

Within the present invention the term "whole broth" is to be understood to consist of or to contain a partly or completely fermented medium and may even contain components of the original medium but also any compound generated during the fermentation process. "Whole broth" may also contain part of or all of the microbial biomass of the fermentation microorganism.

Within the present invention the term "whole broth enzyme composition" is to be understood as any whole broth as defined herein containing at least one enzyme. The at least one enzyme may have been added to the whole broth, may have been part of the original fermentation medium but may also be generated during the production process according to the present invention. "Whole broth enzyme composition" may also contain a mixture of two or more of such enzymes.

Within the present invention the whole broth enzyme composition preferably contains at least one enzyme belonging to the class of hydrolases. Within a particularly preferred embodiment of the present invention, the whole broth enzyme composition contains at least one enzyme belonging to the class of hydrolases which has been produced by the at least one filamentous fungus cell. Within another also particularly preferred embodiment, the whole broth enzyme composition contains at least one enzyme belonging to the class of cellulases and at least one enzyme belonging to the class of hemicellulases which has been produced by the at least one filamentous fungus cell.

Within the present invention, the term "enzyme belonging to the class of hydrolases" is to be understood as comprising any enzyme, capable of the hydrolysis of a chemical bond. Enzymes belonging to the class of hydrolases are classified as EC 3 in the EC number classification of enzymes. According to the present invention, the term "hydrolases" comprises cellulases, hemicellulases and may also encompass pectinases, oxydases and accessory proteins.

As used within the present invention, the term "cellulase" refers to any enzyme capable of hydrolyzing cellulose polymers to shorter oligomers and/or glucose. Cellulases preferred within the whole broth enzyme composition include cellobiohydrolases (CBH) (EC 3.2.1.-), endo-1,4-β-glucanases (EG) (EC 3.2.1.4).), beta-glucosidase (EC 3.2.1.4), cellobiose hydrolase (EC 3.2.1.21), glycoside hydrolase 61 (GH61 and CBM33).

As used within the present invention, the term "hemicellulase" refers to any enzyme capable of degrading or supporting the degradation of hemicellulose. Hemicellulases preferred within the whole broth enzyme composition include β-glucanases (EC 3.2.1.-), endo-xylanases (EC 3.2.1.8), β-xylosidases (EC 3.2.1.37), acetylxylan esterase (EC 3.1.1.72), acetylgalactan esterase (3.1.1.6), acetyl mannan esterase, feruloyl esterase (EC 3.1.1.73), glucuronoyl esterase (EC 3.1.1.-), α-L-arabinofuranosidase (EC 3.2.1.55), α-arabinopyranosidase (3.2.1.-), α-galactosidase (EC 3.2.1.22), β-gaiactosidase (EC 3.2.1.23), α-glucuronidases (EC 3.2.1.139), β-mannase (EC 3.2.1.78), β-mannosidases (EC 3.2.1.25), mannan 1,4-mannobiosidase (EC 3.2.1.100), arabinogalactan endo-beta-1,4-galactanase (EC 3.2.1.89), endo-beta-1,3-galactanase (EC 3.2.1.90), galactan endo-beta-1,3-galactanase (EC 3.2.1.181, glucuronoarabinoxylan endo-1,4-beta-xylanase (EC 3.2.1.136), alpha-L-fucosidase (EC 3.2.1.51), coniferin beta-glucosidase (EC 3.2.1.126), xyloglucan hydrolases (EC 3.2.1.150, 151, 155), xylan α-1 ,2-glucuronosidase (EC 3.2.1.131), endo-xylogalacturonan hydrolase (EC 3.2.1.-; GH28), α-amylase (EC 3.2.1.1 ), glucan 1 4-α-glucosidase (EC 3.2.1.3), galactan 1,3-galactosidase (GH43), -1,4,-endogalactanase (EC 3.5.1.89; GH53), α-rhamnosidase (EC 3.2.1.40), β-rhamnosidase (EC 3.2.1.43).

As used within the present invention, the term "pectinase" refers to any enzyme capable of degrading or supporting the degradation of pectin. Pectinases preferred within the whole broth enzyme composition include polygalacturonases (EC 3.2.1.15, 67, 82; GH28pectin methyl esterase (EC 3.1.1.11), pectin acetyl esterase (EC 3.1.1.- ), rhamnogalacturonase (EC 3.2.1.-; GH28), rhamnogalacturonan acetylesterase (EC 3.1.1.86), rhamnogalacturonan galacturonohydrolase (EC 3.2.1.-), xylogalacturonan hydrolase (EC 3.2.1.-), pectin methylesterase (EC 3.1.1.11), beta-arabinofuranosidase (EC 3.2.1.55), beta-1 ,4-galactanase (EC 3.2.1.89), beta-1 ,3-galactanase (EC 3.2.1.90), beta-galactosidase (EC 3.2.1.23), alpha-galactosidase (EC 3.2.1.22), feruloyl acetyl esterase (EC 3.1.1.-), alpha-fucosidase (EC 3.2.1.51), (beta-fucosidase) (EC 3.2.1.38), beta-apiosidase (EC 3.2.1.-), alpha-rhamnosidase (EC 3.2.1.40), beta-rhamnosidase (EC 3.2.1.43), alpha-arabinopyranosidase (EC 3.2.1.-), beta-glucuronidase (EC 3.2.1.31), alpha-glucuronidase (EC 3.2.1.139), beta-xylosidase (EC 3.2.1.37) and alpha-xylosidase (EC 3.2.1.x).

As used within the present invention the term "accessory protein" refers to any enzyme capable of supporting cellulolytic enzyme activity. The term is well known to a person skilled in the art. Preferred accessory proteins within the whole broth enzyme composition include Expansin, Swollenin, Loosinin and CIP Proteins (EC 3.1.1.-; CE15).

As used within the present invention, the term "oxidative enzymes" refers to any enzyme capable of catalyzing an oxidation reaction. Oxidative enzymes preferred within the whole broth enzyme composition include lytic polysaccharide monooxygenase (LPMO) (AA9-11; previously GH61 and CBM33, resp.) (EC 1.14.99.53-56, 1.14.99.B10), lignin peroxidase (EC 1.11.1.14), manganese peroxidase (EC 1.11.1.13), aryl-alcohol oxidase (EC 1.1.3.7), glyoxal oxidase (EC 1.1.3.), carbohydrate oxidases (EC 1.1.3.4, 9, 10), cellobiose dehydrogenase (EC 1.1.99.18), catalase (hydrogen-peroxide oxidoreductase) (EC 1.11.1.6 or EC 1 .11.1.21), dye-decolorizing peroxidase (EC 1.11.1.19), laccase (EC 1.10.3.2), peroxidase (EC 1.11.1.x) and versatile peroxidase (EC 1.11.1.16).

The enzymes referenced within the present invention are classified according nomenclatures that are either based on the International Union of Biochemistry and Molecular Biology's Enzyme Nomenclature and Classification (http://www.chem.qmul.ac.uk/iubmb/enzyme/) or on Carbohydrate-Active EnZYmes (http://www.cazy.org/) database.

Within the present invention, the at least one enzyme belonging to the class of hydrolases amounts preferably to from 1 to 45 wt.-% (relative to the weight of the whole broth enzyme composition), further preferred to from 1 to 25 wt.-%, particularly preferred to from 1 to 20 wt.-%, also preferred to from 2 to 15 wt.-%, from 2 to 14 wt.-%, from 3 to 12 wt.-% and most preferred to from 5 to 11 wt.-%.

Within the present invention the term "fermentation medium originating from hydrolysis of lignocellulosic biomass" can be any medium which has been prepared by chemical, mechanical and/or enzymatic hydrolysis of lignocellulosic biomass material and preferably comprises prior mechanical and/or acidic pretreatment of the lignocellulosic biomass. Within a preferred embodiment of the inventive process, the hydrolysis has been carried out by mechanical and enzymatical hydrolysis or by sole enzymatic hydrolysis without the addition of any organic and/or inorganic acid(s). The hydrolysis of lignocellulosic biomass is known to a person skilled in the art, exemplary methods are for example described within Vishnu et al. 2012 (Trends in bioconversion of lignocellulose: Biofuels, platform chemicals & biorefinery concept in bioconversion of lignocellulose: Biofuels, platform chemicals & biorefinery concept. Progress in Energy and Combustion Science, August 2012, vol. 38 (4), 522-550) and Prasad et al. 2019 (Bioethanol production from waste lignocelluloses: A review on microbial degradation potential Chemosphere Volume 231, September 2019, p. 588-60).

Within the present invention the term "lignocellulose-containing biomass" is to be understood to comprise all kind of biomass known to a person skilled in the art as comprising lignocellulose. Particularly preferred lignocellulose-containing biomass according to the present invention include wood, cereal straw such as but not limited to wheat straw, rice straw, barley stray, rye straw and oat straw, and/or husks and/or brans thereof, bagasse, oat hulls, switch grass, cellulose, raw paper pulp (obtained from pulp and paper production) and mixtures thereof. Additional components may comprise one or more of the following components: purified cellulose, pulp, milk whey or molasses. Lignocellulosic biomass which is particularly suitable for hydrolysis according to the process of the present invention is selected from the group consisting of cereal straw, cereal bran, cereal husks, wood, bagasse and mixtures thereof.

In a preferred embodiment the lignocellulose-containing material contains at least 25 wt.-%, preferably at least 40 wt.-%, more preferably at least 70 wt.-%, even more preferably at least 80 wt.-% and most preferred at least 90 wt.-% lignocellulose. It is to be understood that the lignocellulose-containing material may also comprise other compounds such as proteinaceous material, starch, sugars, such as fermentable sugars and/or non-fermentable sugars.

Within the process of the present invention, the fermentation medium contains from 5 to 450 g/L glucose and from 2 to 300 g/L xylose, wherein glucose contents from 5 to 420 g/L, from 8 to 400 g/L and from 10 to 280 g/L are preferred and wherein xylose contents from 3 to 280 g/L, from ¹3 to 270 g/L and from 4 to 260 g/L are preferred. Further preferred ranges of glucose are from 10 to 450 g/L, from 40 to 400 g/L and from 50 to 350 g/L whereas further preferred ranges of xylose are from 10 to 280 g/L, from 30 to 250 g/L and from 50 to 220 g/L. Further preferred is a ratio from glucose to xylose selected from the range of from 5 to 1, such as a ratio selected from the range of from 3 to 1, from 4.0 to 1.5, of from 3.5 to 1.5 or of from 3.0 to 1.5. Ratios selected from the range of from 2.5 to 1.0 are most preferred as a maximum of glucose and xylose have been released from the lignocellulosic biomass during hydrolyzation enabling a higher yield of whole broth enzyme composition.

The fermentation medium originating from hydrolysis of lignocellulosic biomass has a high density of from 0.90 to 2.00 kg/L, preferably of from 0.95 to 1.90 kg/L, further preferred of from 1.00 to 1.50 kg/L and most preferred of from 1.05 to 1.35 kg/L.

The fermentation medium originating from hydrolysis of lignocellulosic biomass has a dry matter content of from 10 to 75 wt.-%, preferably of from 10 to 70 wt.-%, further preferred of from 20 to 65 wt.-%, from 30 to 65 wt.-% or from 40 to 60 wt.-% whereas a dry matter content of from 10 to 20 wt.-% and from 10 to 15 wt.-% is also preferred.

The "providing" of the fermentation medium according to step (a) of the inventive process can be carried out by any method and within any means known to a person skilled in the art as suitable for the inventive process. Within a preferred embodiment the fermentation medium originating from hydrolysis of lignocellulosic biomass is provided within a batch or fed batch reactor which is preferred equipped with a stirring device and a cooling device.

Within a preferred embodiment of the inventive process, the fermentation medium has a furfural content of less than 0.5 g/L, preferably less than 0.2 g/L, further preferred less than 0.1 g/L, also preferred less than 0.05 g/L and is most preferred selected from the range of from 0.001 mg/L to 0.5 g/L or from 0.01 mg/L to 0.25 g/L.

Within another preferred embodiment of the inventive process, the fermentation medium has a hydroxymethyl furfural content of less than 0.5 g/L, preferably less than 0.2 g/L, further preferred less than 0.1 g/L, also preferred less than 0.05 g/L and is most preferred selected from the range of from 0.001 mg/L to 0.5 g/L or from 0.01 mg/L to 0.25 g/L.

Within another preferred embodiment from 0.05 to 5 wt.-% nitrogen are added during step (a) and/or (b) of the inventive process. Further preferred ranges are from 0.08 to 5 wt.-%, from 0.1 to 5 wt.-% and from 0.5 to 5 wt.-%. The nitrogen can be added in any form known to a person skilled in the art as suitable for the inventive purpose and may be added in form of ammonium sulfate, ammonia, urea or combinations thereof. The amount of nitrogen can be added by feeding or by adding the total amount to the fermentation medium at any time during step (a) and/or (b) of the inventive process. It is thereby preferred that the nitrogen is added as a 25% (wt.-/wt.) solution of ammonia or a 40 % (wt./wt.) solution of urea.

Within another preferred embodiment from 0.5 to 350 mg/L FeSO₄, MnSO₄, MgSO₄ and/or ZnSO₄ are added during step (a) and/or (b) of the inventive process. The amount of FeSO₄, MnSO₄, MgSO₄ and/or ZnSO₄ can be added by feeding or by adding the total amount to the fermentation medium at any time during step (a) and/or (b) of the inventive process. It is thereby preferred that FeSO₄ is added in an amount of from 0.5 to 35 mg/l and MgSO₄ is added in an amount of from 200 to 350 mg/l.

Within a particularly preferred embodiment of the inventive process no mono- and/or disaccharides, in particular no glucose, fructose or xylose, are added to the fermentation medium originating from hydrolysis of lignocellulosic biomass at any time during the inventive process.

Within a preferred embodiment of the inventive process the pH of the fermentation medium has been adjusted to a pH selected from the range of from pH 2.0 to pH 6.0, wherein ranges of from pH 3.0 to 5.5 and from pH 3.5 to 5.5 as well as from pH 3.5 to 5.0 are particularly preferred. The adjusting of the pH can be carried out by any means and method known to a person skilled in the art as suitable for the inventive purpose. Within the process of the present invention the pH is preferably adjusted by addition of an acid such as sulfuric acid or acetic acid, NaOH, H₃PO₄ or ammonia.

Within a preferred embodiment of the inventive process the process further comprises step (ai) concentration of the fermentation medium by evaporation, membrane filtration, thin layer evaporation, falling-film or downstream evaporation to decrease the weight of the fermentation medium by factor 2 to 6, wherein a decrease of weight of the fermentation medium by a factor of 2.5 to 6, 3 to 6, 3.5 to 6 and 4 to 6 is also preferred. The decrease of weight of the fermentation medium is thereby mostly achieved due to a decrease of water content of the original fermentation medium. It is thereby possible to achieve the decrease of weight of the fermentation medium by concentration of only part of the medium and blending the concentrated and non-concentrated medium before carrying out step (b) or by concentration of the complete fermentation medium to the desired end content, desired weight decrease, respectively.

Within a further preferred embodiment of the inventive process the fermentation medium originating from lignocellulosic biomass has a content of organic acids of less than 5 wt.-%, a content of inorganic acids of less than 6 wt.-%, a content of inorganic salts of less than 3 wt.-% and/or an arabinose content of less than 1 wt.-%, wherein the following contents are particularly preferred: 0.5 to 2.5 wt.-% organic acids, 0.5 to 5 wt.-% inorganic acids, 0.2 to 2.5 wt.-% inorganic salts and/or 0.05 to 0.75 wt.-% arabinose. It is thereby also particularly preferred that the content of water soluble chloride is below 0.5 wt.-%, the content of acetic acid is below 35 g/L and/or the content of Na-D/L-lactate is below 15 g/L.

According to step (b) of the inventive process, at least one filamentous fungus cell wherein SEQ ID NO: 1 has been mutated by insertion of the single nucleotide polymorphism (SNP) A149G is added to the fermentation medium. The addition can be carried out by any means and measure known to a person skilled in the art as suitable for the inventive process. Within a preferred embodiment, the at least one filamentous fungus cell is added in a quantity of from 10² to 10¹⁰ cells, preferably in a quantity of from 10³ to 10⁸ cells and most preferred in a quantity of from 10⁴ to 10⁷ cells per g of fermentation medium. The at least one filamentous fungus cell can thereby be added in dried form, as conidia or in form of a preculture, containing rest of preculturing medium. It is also possible to add the at least one filamentous fungus cell in form of a fully cultured medium (also referred to as main culture).

Within the present invention the term "filamentous fungus cell" is to be understood as any cell from any filamentous fungus existing in nature and/or known to a person skilled in the art. The term also comprises any filamentous fungus cell either of natural origin or modified. The term "modified" refers to genetically and non-genetically modified fungi. i.e. fungi which have been modified by genetic methods (e.g. transformation) and non-genetic methods e.g. chemical mutagenesis or irradiation, both of which are known to those skilled in the art. Within a preferred embodiment the at least one filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes,* wherein *Trichoderma* and *Aspergillus* are particularly preferred, most preferred is *Trichoderma reesei* (teleomorph: *Hypocrea jecornia*)*.* Within another preferred embodiment of the present invention, the at least one filamentous fungus cell is a genetically modified filamentous fungus cell with the with the ability to express at least one heterologous hydrolase enzyme, at least one heterologous pectinase enzyme, at least one heterologous oxidative enzyme and/or at least one heterologous accessory protein, preferably an enzyme belonging to the class of beta-glucosidases, to the class of xylanase enzymes, to the class of beta-xylosidase enzymes and/or to the class of lytic polysaccharide monooxygenase enzymes.

Within such a preferred embodiment, the at least one heterologous hydrolase enzyme preferably originates from another filamentous fungus such as - but not limited to *- Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.* Within a particularly preferred embodiment the at least one filamentous fungus cell is a *Trichoderma reesei* cell and the at least one heterologous hydrolase enzyme originates from *Acremonium, Ajellomyces, Alternaria, Armillaria, Arthroderma, Aspergillus, Bionectria, Bipolaris, Ceriporiopsis, Chaetomium, Cladophialophora, Clohesyomyces, Colletotrichum, Coniochaeta, Coniosporium, Diaporthe, Dothistroma, Emericella, Epicoccum, Exophiala, Fomes, Fonsecaea, Fusarium, Gibberella, Grosmannia, Hebeloma, Hortaea, Humicola, Hypocrea, Hypoxylon, Irpex, Isaria, Kuraishia, Leucoagaricus, Madurella, Magnaporthe, Marssonina, Metarhizium, Moniliophthora, Myceliophthora, Mycosphaerella, Neurospora, Oidiodendron, Ophiostoma, Paecilomyces, Paraphaeosphaeria, Penicillium, Phanerochaete, Phialophora, Pleurotus, Pochonia, Pseudocercospora, Pseudogymnoascus, Pyrenophora, Rasamsonia, Rhinocladiella, Rhizopus, Rhizosphaera, Rhynchosporium, Setosphaeria, Sphaerulina, Sporothrix, Stachybotrys, Stemphylium, Talaromyces, Termitomyces, Tilletiaria, Torrubiella, Trametes, Trichoderma, Trichophyton, Uncinocarpus* and/or *Valsa* species.

According to the present invention, the at least one filamentous fungus cell is a filamentous fungus cell wherein the A at position 149 of SEQ ID NO: 1 has been changed to a G. The change can thereby be carried out by any means and measure known to the person skilled in the art as suitable for the purpose.

Within the present invention SEQ ID NO:1 and its changed sequence SEQ ID NO:11 are defined within the sequence protocol.

Mixing according to step (c) of the process of the present invention is carried out for a time period from 1 minute to 10 days, preferably from 10 hours to 7 days, further preferred from 24 hours to 5 days, preferably under constant stirring with a power input from 150 to 20000 W/ m³ and more preferably from 500 to 15000 W/m³ and under oxygen controlled conditions. The average dissolved oxygen level is preferably selected from 0.01% to 80%, preferred from 0.1% to 50%, particularly preferred from 5% to 30% and most preferred from 12% to 28%. Within a particularly preferred embodiment, the dissolved oxygen level is controlled by a stirrer or compressed air flow or internal reactor pressure or a combination of two or three of these measures. Furthermore, mixing according to step (c) of the inventive process is carried out at a temperature of from 20 to 35 °C, preferably at a temperature of from 21 to 34 °C wherein a temperature selected from the range of from 22 to 33 °C is also preferred.

"Mixing" according to step (c) of the process of the present invention is preferably conducted in a batch mode (discontinuous), in the fed-batch mode or in a continuous mode. Most preferably, the inventive process is conducted in the batch mode.

"Obtaining" according to step (d) of the inventive process is preferably carried out by harvesting the whole fermentation broth at the end of the time period applied for mixing during step (c) as it is without further treatment. It is, however, also possible within an alternative embodiment to practice a solid-liquid-separation according to step (e) of the inventive process. The solid-liquid-separation according to step (e) is carried out by any measure known to a person skilled in the art as suitable for the inventive purpose such as but not limited to filtration, pressing, membrane separation, flotation, precipitation, decantation and centrifugation or combinations thereof. Preferred are filter-based solid-liquid separations. It is further particularly preferred to use a filter press. The residues after the filtration should have a minimal solid content of 20 % (wt./wt.), preferably 25 % (wt./wt.), particularly preferred 30 % (wt./wt.) and most preferred 40 % (wt./wt.) solid content. Another method for the separation according to step (e) is centrifugation by e.g. using a decanter. In case the process according to the present invention involves solid-liquid-separation, the whole broth enzyme composition obtained according to step (d) of the inventive process is considered to be the liquid fraction.

Within a preferred embodiment of the inventive process, the process further comprises step
(aii) sterilization of the fermentation medium according to step (a) or the concentrated fermentation medium according to step (ai).

Sterilization can thereby be carried out by any means or measure known to a person skilled in the art as suitable for the inventive purpose. Within a preferred embodiment, sterilization is carried out by filtration, such as but not limited to membrane filtration processes or by ultra high temperature heating. A combination of two or more sterilization methods is also possible, however, it is particularly preferred to only apply ultra high temperature heating (also referred to as UHT). The UHT treatment is preferably carried out at a temperature of from 100 to 155 °C and for a duration of from 10 to 30 seconds, more preferred at a temperature of from 120 to 140 °C for a duration of from 10 to 20 seconds.

Within another aspect, the present invention relates to a filamentous fungus cell wherein the A at position 149 of SEQ ID NO:1 has been changed to a G. The change of SEQ ID NO:1 can be carried out by any means and measure known to a person skilled in the art to be suitable for the inventive purpose. The changed sequence SEQ ID NO:1 is listed in form of SEQ ID NO:11. The term "filamentous fungus cell" has been defined within the description. All definitions given apply.

Within a preferred embodiment, the filamentous fungus cell is a genetically modified filamentous fungus cell with the ability to express at least one heterologous hydrolase enzyme. Within a particularly preferred embodiment, the filamentous fungus cell contains at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence. The respective heterologous enzyme sequence may originate from any fungus or microorganism known to a person skilled in the art as suitable for the inventive purpose. Within a preferred embodiment the heterologous enzyme sequence originates from another species of filamentous fungus.

In another aspect the present invention relates to a whole broth enzyme composition prepared according to the process as defined before.

In a further aspect the present invention relates to the use of a whole broth enzyme composition as defined before for the hydrolyzation of lignocellulosic biomass. The definition within the description for "hydrolyzation" and "lignocellulosic biomass" apply.

### Generally preferred embodiments

In the following, generally preferred embodiments of the present invention are listed which do not limit the scope of the invention and/or scope of the claims in any respect. The generally preferred embodiments illustrate particularly suitable embodiments for the production of whole broth enzyme composition by the filamentous fungus *Trichoderma reesei.*

### Generally preferred embodiment 1

Process for production of a whole broth enzyme composition, comprising the following steps:
(a) providing a fermentation medium, originating from hydrolysis of lignocellulosic biomass, with a glucose content of from 40 to 400 g/L, a xylose content of from 50 to 200 g/L, a density of from 1.05 to 1.35 kg/L and a dry matter content of from 30 to 65 wt.-%;
(b) addition of at least one *Trichoderma reesei* cell wherein the A at position 149 of SEQ ID NO:1 has been changed to a G;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment 2

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 40 to 400 g/L, a xylose content of from 50 to 200 g/L, a density of from 1.05 to 1.35 kg/L, a dry matter content of from 30 to 65 wt.-%, a nitrogen content of from 0.05 to 5 wt.-%, a MgSO₄ content of from 200 to 350 mg/L and a average dissolved oxygen level of from 5 to 30%;
(b) addition of at least one *Trichoderma reesei* cell wherein the A at position 149 of SEQ ID NO:1 has been changed to a G;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment 3

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 40 to 400 g/L, a xylose content of from 50 to 200 g/L, a density of from 1.05 to 1.35 kg/L, a dry matter content of from 30 to 65 wt.-%, a nitrogen content of from 0.05 to 5 wt.-%, a MgSO₄ content of from 200 to 350 mg/L, a average dissolved oxygen level of from 5 to 30% and a pH of from 3.5 to 5;
(b) addition of at least one *Trichoderma reesei* cell wherein the A at position 149 of SEQ ID NO:1 has been changed to a G;
(c) mixing of the fermentation medium and the at least one *Trichoderma reesei* cell for a time period of from 1 min to 10 days at a temperature of from 20 to 35 °C;
(d) obtaining a whole broth enzyme composition containing at least one enzyme belonging to the class of cellulases and at least one enzyme belonging to the class of hemicellulases which has been produced by the at least one *Trichoderma reesei* cell.

### Generally preferred embodiment 4

Process for production of a whole broth enzyme composition, comprising the following steps:
(a) providing a fermentation medium, originating from hydrolysis of lignocellulosic biomass, with a glucose content of from 5 to 25 g/L, a xylose content of from 2 to 15 g/L, a density of from 1.05 to 1.35 kg/L and a dry matter content of from 30 to 65 wt.-%;
(b) addition of at least one *Trichoderma reesei* cell wherein the A at position 149 of SEQ ID NO:1 has been changed to a G;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment 5

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 5 to 25 g/L, a xylose content of from 2 to 15 g/L, a density of from 1.05 to 1.35 kg/L, a dry matter content of from 30 to 65 wt.-%, a nitrogen content of from 0.05 to 5 wt.-%, a MgSO₄ content of from 200 to 350 mg/L and a average dissolved oxygen level of from 5 to 30%;
(b) addition of at least one *Trichoderma reesei* cell wherein the A at position 149 of SEQ ID NO:1 has been changed to a G;
(c) mixing of the fermentation medium and the at least one *Trichoderma reesei* cell for a time period of from 1 min to 10 days at a temperature of from 20 to 35 °C;
(d) obtaining a whole broth enzyme composition.

### Generally preferred embodiment 6

Process for production of a whole broth enzyme composition, comprising the following steps:
(a) providing a fermentation medium, originating from hydrolysis of lignocellulosic biomass selected from wheat straw, barley straw, oat straw, rice straw, rye straw, bagasse or corn stover; with a glucose content of from 5 to 25 g/L, a xylose content of from 2 to 15 g/L, a density of from 1.05 to 1.35 kg/L, a dry matter content of from 30 to 65 wt.-%, a nitrogen content of from 0.05 to 5 wt.-%, a MgSO₄ content of from 200 to 350 mg/L, an average dissolved oxygen level of from 5 to 30% and a pH of from 3.5 to 5;
(b) addition of at least one *Trichoderma reesei* cell wherein the A at position 149 of SEQ ID NO:1 has been changed to a G;
(c) mixing of the fermentation medium and the at least one *Trichoderma reesei* cell for a time period of from 1 min to 10 days at a temperature of from 20 to 35 °C;
(d) obtaining a whole broth enzyme composition containing at least one enzyme belonging to the class of cellulases and at least one enzyme belonging to the class of hemicellulases which has been produced by the at least one *Trichoderma reesei* cell.

### Generally preferred embodiment 7

Process for production of a whole broth enzyme composition as defined by any of generally preferred embodiments 1 to 6, wherein the *Trichoderma reesei* cell is further genetically modified by genetic methods (e.g. transformation) and/or non-genetic methods e.g. chemical mutagenesis or irradiation and wherein this further genetically modified *Trichoderma reesei* cell is able to express at least one heterologous hydrolase enzyme, at least one heterologous pectinase enzyme, at least one heterologous oxidative enzyme and/or at least one heterologous accessory protein.

### Generally preferred embodiment 8

*Trichoderma reesei* cell wherein the A at position 149 of SEQ ID NO:1 has been changed to a G and wherein the *Trichoderma reesei* cell is a genetically modified *Trichoderma reesei* cell, wherein the *Trichoderma reesei* cell comprises at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence.

### Generally preferred embodiment 9

*Trichoderma reesei* cell as defined by generally preferred embodiment 8, wherein the at least one heterologous beta-glucosidase enzyme encoding sequence originates from *Cladophialophora* species, *Pseudocercospora* species and/or *Talaromyces* species and wherein the at least one xylanase enzyme encoding sequence originate from *Fomes* species, wherein the at least one beta-xylosidase encoding enzyme sequence originates from *Aspergillus* species and wherein the at least one lytic polysaccharide monooxygenase enzyme encoding sequence originates from *Aspergillus* species, *Trichoderma* species or *Hypocrea* species.

### Generally preferred embodiment 10

Whole broth enzyme composition produced according to a process as defined by any of generally preferred embodiments 1 to 7 containing at least one heterologous betaglucosidase enzyme produced by the genetically modified *Trichoderma reesei* cell as defined by generally preferred embodiment 8 or 9 and containing whole of or part of these *Trichoderma reesei* cell.

### Generally preferred embodiment 11

Use of a genetically modified Trichoderma reesei cell as defined by generally preferred embodiment 8 or 9 for the production of whole broth enzyme composition as defined by generally preferred embodiment 10 for the hydrolyzation of lignocellulosic biomass.

### Figures and examples

The present invention is described by the following figures and examples. It is thereby emphasized that the figures and examples do not limit the scope of the invention and claims but merely constitute further illustration of the invention, inventive purpose and benefits achieved by the inventive method.

### List of figures

- Figure 1:: Protein concentrations in the culture supernatants of pSEQ1M-HygR transformants MSEQ1-1 to -3 and reference strain M18.2b. Values are given in relation to the average protein concentration in the supernatants of the host strain M18.2b which is set to 1.
- Figure 2:: Biomass concentrations in the culture supernatants of pSEQ1M-HygR transformants MSEQ1-1 to -3 and reference strain M18.2b. Values are given in relation to the average biomass concentration in the supernatant of the host strain M18.2b which is set to 1.

### General

The examples describe the mutation of the *Trichoderma reesei* SEQ ID NO:1 gene by insertion of a single nucleotide polymorphism (SNP) and show the effect of that SEQ ID NO:1 mutation on the protein production and biomass formation of T. *reesei.*

Standard methods known to those skilled in the art and described e.g. by Sambrook and Russel (Molecular Cloning - A laboratory manual; Cold Spring Harbor Laboratory Press, New York) or by Jansohn et al. (Gentechnische Methoden, Elsevier, München) were used for DNA agarose gel electrophorese, purification of DNA, transformation of *Escherichia coli,* plasmid propagation and purification, amplification of pieces of DNA by polymerase chain reaction (PCR) and isolation of genomic DNA from *Trichoderma reesei.* Ligation-independent cloning (LIC) was done essentially as described by Aslanidis and de Jong (1990, Nucleic Acid Res. 18 (20), 6069).

### Example 1: Construction of a SEQ ID NO: 1 mutation vector

Plasmid pSEQ1M (SEQ ID NO: 2) that contains the flanking regions for introduction of the mutation A149G (position according to SEQ ID NO: 1) into the SEQ ID NO:1 gene and a LIC site for insertion of the marker gene cloned into a pUC19-derived plasmid was synthesized by Thermo Fisher Scientific.

Plasmid pSEQ1M was digested with *Srf*I (New England Biolabs) according to the manufacturer's instructions and purified using the Wizard PCR purification kit from Promega.

The hygromycin B resistance cassette (HygR) (SEQ ID NO: 3) was synthesized by Thermo Scientific. HygR was amplified by PCR using the DNA from Thermo Scientific as template, primers SEQ1 MHygRfw (5'- AACAAGACACAGCCCTATAAC -3'; SEQ ID NO: 4) and SEQ1 MHygRrv (5'- AACAGACAAGAGCCCTATAAC -3'; SEQ ID NO: 5) and phusion polymerase from Thermo Fisher Scientific according to the manufacturer's instructions (annealing temperature: 68.5 °C, elongation time: 40 sec, 30 cycles). The amplicon (2.4 kb) was purified using the Wizard PCR purification kit from Promega.

The PCR-amplified HygR marker was fused with linearized pSEQ1M using ligation independent cloning (LIC). The linearized vector was treated with T4 DNA polymerase in the presence of dTTP. The PCR-amplified HygR marker gene was treated with T4 DNA polymerase in the presence of dATP. T4 DNA polymerase treated vector and marker gene were mixed and annealed as described in the cited literature. The assay was then transformed in chemically competent *Escherichia coli* XL1-Blue cells (Agilent), plated on LB-Agar plates containing 100 mg.l⁻¹ ampicillin (LB-Amp) and incubated at 37 °C for 24 h. Colonies were picked from the agar plates using toothpicks, transferred into liquid LB-Amp medium and incubated at 37 °C for 24 h with shaking (250 RPM). Plasmid DNA was isolated and integration of the insert was verified by digestion with *Pm*el*.* Plasmid clones were verified by Sanger sequencing and one plasmid with correct sequence was designated pSEQ1M-HygR.

### Example 2: Transformation of the SEQ ID NO: 1 mutation vector into Trichoderma reesei

Vector pSEQ1M-HygR was digested with *Pm*el (New England Biolabs) according to the manufacturer's instructions and the mutation cassette (5.9 kb) was purified by agarose gel electrophoresis and with the Wizard PCR purification kit from Promega. *Trichoderma reesei* M18.2b (DSM 19984) was transformed with the digested vector essentially as described in Penttilä et al (1987) Gene 61: 155-164 or Gruber et al (1990) Curr Genet 18: 71-76. The transformants were selected on potato dextrose agar plates containing 100 mg.l⁻¹ of hygromycin and 1 M sorbitol and purified by singularisation. Conidia stocks of the purified strains were prepared by growing them on potato dextrose agar plates at 30 °C until the plates were covered with spores. The conidia were harvested with sterile sodium chloride (0.9 g.l⁻¹)-Triton X-100 (0.01 g.l⁻¹) solution, adjusted to OD₆₀₀ = 10, supplemented with 50 g.l⁻¹ of glycerol and stored at -80 °C.

Genomic DNA was isolated from the mycelium of the transformants and the host strain. The integration of the SEQ ID NO:1 mutation cassette at the intended locus was verified by PCR using phusion polymerase from Thermo Fisher Scientific according to the manufacturer's instructions, genomic DNA from the transformants as template and primers SEQ1MKOfw (5'- GATGGCTGTGTAGAAGTAC -3'; SEQ ID NO: 6) and SEQ1MKOrv (5'- GAGAGGTTTGACTGGATC -3'; SEQ ID NO: 7) (annealing temperature: 57.2 °C, elongation time: 1 min 25 sec, 30 cycles). A 2.4 kb band with primers SEQ1 MKOfw and SEQ1 MKOrv indicates the integration of the mutation cassette at the SEQ ID NO:1 locus. Genomic DNA from strain M18.2b was also tested as a control. In order to verify that the intended mutation had been inserted into the *SEQ1* ORF, the respective region was amplified by PCR using phusion polymerase from Thermo Fisher Scientific according to the manufacturer's instructions, genomic DNA from the transformants as template and primers SEQ1MSeqfw (5'- TGACATTCTCCTGGACACCC -3'; SEQ ID NO: 8) and SEQ1MSeqrv (5'- GTTGCGTCTTCTCTTGCGTC -3'; SEQ ID NO: 9) (annealing temperature: 64.5 °C, elongation time: 30 sec, 30 cycles). The 0.9 kb amplicon was purified using the Wizard PCR purification kit from Promega and sequenced using Primer MSeq-01 (5'- AAGCATTGACGACAGAAAGG -3'; SEQ ID NO: 10).

Three strains containing the mutation from pSEQ1M-HygR in the SEQ1 ORF were named MSEQ1-1 to -3.

### Example 3: Growth of the SEQ ID NO: 1 mutation strains in shake flasks

The strains MSEQ1-1 to -3 and M18.2b were grown in shake flasks in hydrolysate medium 1. Hydrolysate medium 1 contains (g.l⁻¹):

| Name | Concentration [g/l] |
|---|---|
| Acetic acid | 0.34 |
| Calcium | 0.12 |
| Chloride, water soluble | 0.15 |
| Copper | 0.0001 |
| Fat (HCI soluble) | 0.001 |
| Furfural | 0.003 |
| Glucose | 6.5 |
| Glycerol | 0.009 |
| HMF | 0.006 |
| Iron | 0.004 |
| Magnesium | 0.048 |
| Manganese | 0.002 |
| Na-D/L-Lactat | 0.097 |
| Nitrogen, soluble | 0.85 |
| Phosphorus | 0.48 |
| Phthalate | 8.2 |
| Potassium | 3.2 |
| Sodium | 0.015 |
| Sulfur | 0.86 |
| Xylose | 3.6 |
| Zinc | 0.001 |

The medium was adjusted to pH 5.5 with HCI or NaOH and sterilized by autoclaving (20 min at 121 °C).

15 ml of the medium were distributed into 50 ml Erlenmeyer shake flasks under a sterile hood. Conidia stocks of strains MSEQ1-1 to -3 and M18.2b were thawed, 75 µl of the conidia suspensions were pipetted into the Erlenmeyer flasks with the medium under a sterile hood and the flasks were closed with rubber foam caps. Three flasks were inoculated per strain. The flasks were incubated at 30 °C with shaking (250 RPM) for 6 days. After 6 days, the cultures were poured into 15 ml tubes. Aliquots were removed, centrifuged (3220xg, 4 °C, 15 min) and the supernatants stored at 4 °C, while the remaining culture broth was used for determination of the biomass (see below).

### Example 4: Characterization of the culture supernatants and broths: Protein concentration, biomass

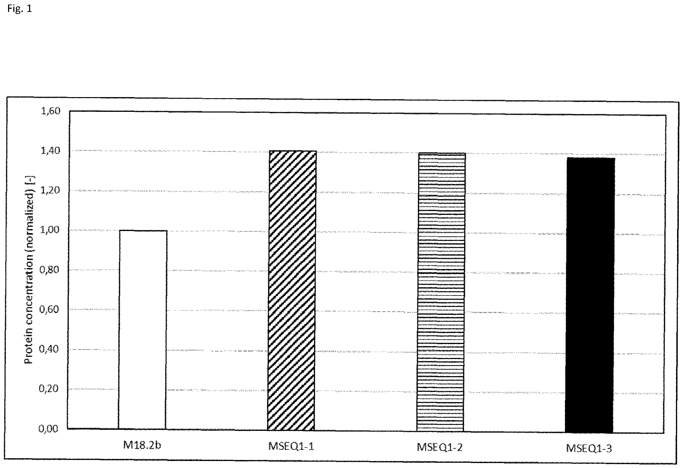

Protein concentrations in the centrifuged culture supernatants of strains MSEQ1-1 to -3 and M18.2b were measured using the Quick Start^{™} Bradford reagent (BioRad) and BSA standard solutions (BioRad) according to the supplier's instructions. The results of the measurements are shown in Figure 1 and are presented in relation to the average protein concentration in the culture supernatants of strain M18.2b, which is set to 1.

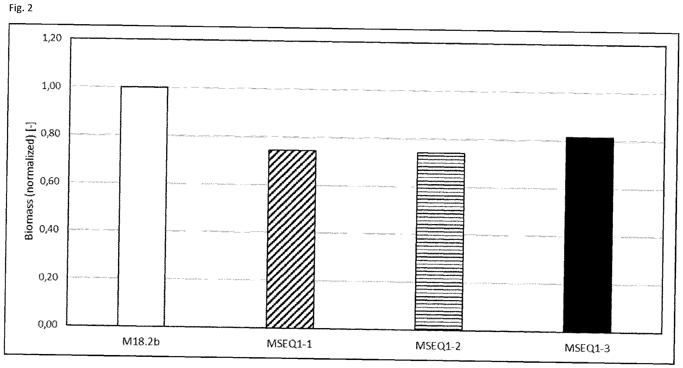

For biomass determination, Whatman^{™} filter discs (P1) were dried at 60 °C until their weight remained constant for 24 h. Culture broths of strains MSEQ1-1 to -3 and M18.2b were filtered using those dried filter discs and the mycelia were washed with at least ten times the broth's volume of deionized water. Then the filter discs with the mycelium were dried at 60 °C until their weight remained constant for 24 h. The filter discs with the dried mycelia were weighted. The biomass concentration in the culture broth was then calculated by subtracting the mass of the dried filter disc from the mass of the dried filter disc with the mycelia and then dividing that value by the volume of the culture broth that had been filtered. The results of the measurements are shown in Figure 2 and are presented in relation to the average biomass concentration in the culture broths of strain M18.2b, which is set to 1.

### Sequence listing

SEQ ID NO: 1
   SEQ1 native gene 
ATGACGCAGTCTCCCATGATCGCCGCGCCGCCCAAGGCCACCAACGAGATCGA
CTGGGTCTCGCCGCTCAAGGCCTACATCCGCGACACCTACGGCGACGACCCCG
AGCGCTATGCCGAAGAGTGCGCCACGCTCAACCGCCTGCGGCAGGACATGCG
CGGCGCCGGCAAGGAGAGCGTCACGGGGAGGGACATGCTCTACCGCTACTAC
GGGCAGCTGGAGCTGCTGGACCTGCGCTTCCCGGTCGATGAGCAGCACATTAA
GATTCCCTTTACATGGTGCGTGACGGAGACACGTTGTGGCCCGCAGCCTTTCTG
TCGTCAATGCTTTGCTTGGAGTTGTGGTAGACGCTGACTTGGGATCTTGTGCTA
GGTTTGACGCATTCACCCACAAACCAACCACGCAGTACTCGCTCGCGTTCGAAA
AGGCCTCTGTCATCTTCAACATCTCCGCCGTCCTTTCCGGCCATGCTGCCATCC
AGAACCGAGAGGATGATTCCGCACTCAAGGTCGCCTACCACTCGTTCCAGGCC
TCGGCCGGCATGTTTACGTACATCAACGAGAACTTCTTGCATGCTCCCTCATTC
GACTTGAGCCGAGAGACCGTCAAGACCTTGATACACATCATGCTCGCCCAGGC
ACAGGAAATCTTTCTGGAGAAGCAGGTCAAGGACCAGAAAAGGCCGGGTTGC
TGGCCAAGCTGGCGTCGCAGTCTGGGTACCTCTATGGACAGGCTGTGGAGGGT
GTCCAGGAGAATGTCACAAAGGCCATCTTTGAGAAGGTCTGGCTGACAATGGTC
CAGGTAAGCTTGGGTGTCTAATGAGGGGATGGATGTTCGCGTGCTTGAGATGC
TGCAGCAGCGAGATTGCTAATTGGGCCGATTGAAACGCCAGATCAAAGCAAGTC
TTCTCAACTCCATGGCGCAATATTATCAGGCAATGGCAGACGACGAAGCGGGC
CAGCATGGCGTGGCCCTATCTCGACTCCAGGTGGCCGATACCCTCGCCAAGGA
CGCCGACCGATTAGCAAAGAGCTTCCCCAGCACCCTACCGTCCAACGCCAATC
TTGGTGCCGACTGCAGTACGCATCTGCAGGAGATTACAAAACGACAGTGCTCGA
 
CGGTGCAGGAACGGCTACGAGAGGCCATCAAGGACAATGACTACATCTACCAT
CAAACCGTCCCCGCGGAGGCGACCCTCCCCCAAATCGCCAAGCTCCCGGCCG
CAAAGCCCATTCCCGTATCGGAGCTCTACGCAGGCCAAGACATCCAGCGCATC
ACGGGGCCCGATTTGTTCTCCAAGATTGTGCCCATGGCCGTTACCGAGTCCGC
CAGCTTATATGACGAGGAAAGGCCAAGCTTGTTAGAGCCGAGACGGAAAAGG
TGGATACGGCAAACGGCGAGATGGCGGCCAGCCTGGACTACCTGCGGCTTCC
GGGGGCGCTGCAGGTGCTCAAGGGCGGGTTCGACCAGGACATTCTTCCCGAC
GAGGATTTCCGGCAATGGTGTGAAGACGTGGCCAACCACGAGAATCCCGTGAG
CATCTTTGACTTTTTGCGGAGCGAGAAGGAGTCGATAGTGTCGACTCTGGACAA
GGCCTCCAAGCAGCTGGACATGGAGGAGAGCGTGTGCGAAAAGATGCGGTCC
AAGTACGAAAACGAATGGAGCCAGCAGCCCAGCGCGCCTCACGACGACCTT
GCGGGGAGACATTCGCAACTACCGGGAGGCCCTGGAGGAGGCCAGCAGGAGC
GACGGCCAGCTGGCGGCGAAGCTGCGCCAGAACGAGACGTGGTTCGACGAGA
TGCGGAACGCCGTCGCAAACGGACAGGTGGACCAGCTCTTTCAAGGGCGGTC
TCCCAGGCCAAGGGGCGAAGCAGCAATGCCGTCAGCCCGTCTGGAAACGAGC
CGAACCTGCTCGATGCAGACTTTGACGAATCTGGGCCTACGGTGGTGGAGCAG
ATTGCAAAGGTCGAAGAGATTCTCAAGAAGCTCAACCTCATCAAGAGAGAGCGG
AATCAGGTCCTCAAGGACCTCAAGGAGAAGGTAAGCTTCTGCTACTGAATCTGC
AGGCTTTATCTCGAAGAGGGCGACATTAACACGGATTGTAGGTCCACAACGACG
ACATCTCTCAGGTCCTCATTCTCAACAAAAAGACGATAGCAAACTATGAGCAGCA
ACTTTTCAAGCAGGAATTGGAAAAGTTTCGGCCGCATCAGAATCGCTTGCTACA
GGCAAACCACAAACAGTCGGCCTTGATGAAGGAGCTCACGGCGACGTTCAACA
CTCTGCTGCAGGACAAGCGCGTGCGCGGAGCAGAGCAAGTACGAATCGATC
CAGCGGCAGAGGCTATCGGCGATTGGCAAGTACAAGCGCGCCTATCAGGAGTT
CTTGGACCTAGAGGCGGGCTTGCAGAGCGCCAAGAACTGGTACTCGGAGATGA
GGGAGACGGTGGAGAGCCTCGAGAAGAACGTGGAGACTTTTGTAAACAACCGG
AGGTCGGAGGGCGCCCAGCTGCTCAACCAGATCGAACAGGAGCGCGCGTCCA
ACAAGAGCCAGCAGGCAGAGCTGGAGAGGAGCGGCTGCGAGGCCTCATGGA
GCGCATGTCGATGGAGCCCGCGCAGCCGGCACCGCCCGCCAGACCACCGTCG
GGAAGACCAACGCCAGCGCCCCTGATGCAGCAGCAGAACCAGGCCTCTCGATA
CGGTCAGGGCGGCAGCAGCAGCAGCAGCAACAACAACAACAACGGCTATCAG
GGGCAGTTTCAGATGCCTACATCGCCGCCGCCCAACCAGCAGAGCTTTACCGG
ATACGCCAGCCCTCCTCCGCAAAGCACCTTTTCGCAACCCGTCTACAATCCGAG
CACGTACGGTAGGAACCCCGGGCCGACATCGCCGCCTCCAAACCAGACATCTT
 
TTAGCATGAATGTCATGAGAGGCCCCCAATCGCCGCCACCCACGCAGACATCG
TTCGGACAGCACCAGCCATATACCATGTACGGGGCATTACCCCAGCAGCAGCA
ACAGCAGCAAACGCAGCAGCAGCAACAACAGCACGCACCTGGAGGATATGTGC
CGCCCGGCTTCGTGCCTCCTCCGCCTCCTCCAGGTCCTCCTCCGCTGGGGCCT
CAGCAGACGATTCATTTTGGGCAGCAGGACTTTGATCCTGCGGTTAGCCACCCG
TCAAGCGCGCAGCCTCGGTCAGCCCAACCGCAGCAGGCGCACGATCCTTGGG
CCGGGTTGAATGCGTGGAAATGA
SEQ ID NO: 2
   pSEQIM 
CTAAATTGTAAGCGTTAATATTTTGTTAAAATTCGCGTTAAATTTTTGTTAAATCAG
CTCATTTTTTAACCAATAGGCCGAAATCGGCAAAATCCCTTATAAATCAAAAGAA
TAGACCGAGATAGGGTTGAGTGGCCGCTACAGGGCGCTCCCATTCGCCATTCA
GGCTGCGCAACTGTTGGGAAGGGCGTTTCGGTGCGGGCCTCTTCGCTATTACG
CCAGCTGGCGAAAGGGGGATGTGCTGCAAGGCGATTAAGTTGGGTAACGCCAG
GGTTTTCCCAGTCACGACGTTGTAAAACGACGGCCAGTGAGCGCGACGTAATA
CGACTCACTATAGGGCGAATTGGCGGAAGGCCGTCAAGGCCTAGGCGCGCCA
GACTGTTTAAACCTAGCACAAGATCCCAAGTCAGCGTCTACCACAACTCCAAGC
AAAGCATTGACGACAGAAAGGCTGCGGCCACAACGTGTCTCCGTCACGCACC
ATGTAAAGGGAATCTTAATGTGCTGCTCATCGACCGGGAAGCGCAGGTCCAGC
AGCTCCAGCTGCCCGTAGTAGCGGTAGAGCATGTCCCTCCCCGTGACGCTCTC
CTTGCCGGCGCCGCGCATGTCCCGCCGCAGGCGGTTGAGCGTGGCGCACTCT
TCGGCATAGCGCTCGGGGTCGTCGCCGTAGGTGTCGCGGATGTAGGCCTTGA
GCGGCGAGACCCAGTCGATCTCGTTGGTGGCCTTGGGCGGCGCGGCGATCAT
GGGAGACTGCGTCATCCTTTTTGTGCGACTTTGGGGCCGCGCGGGTATGGCGG
ACCCGGGGATCTTGGAGGAGGGGGCGGGTTAGGGTGGTCCTGTTGTTGTTCAG
ACGCAAGAGAAGACGCAACGACTTCAATGGCCACGGTCGAGACGGCCGGCAGT
GTGTCGCATGCGCGTCGCCTGCGCGGAGGGACGGGGGGGAACGCTCGGCGG
GTGTGCGGTGCGATGGCAAAGCTGTGCGAGGCGGGAGCTGGCGGCTTGATGA
GAGAGTGCGGAGGATGGGGGGCCCCGTTTGCTGGGATGATCGAGTCTGATC
GATGAAGAGACAGAGGGAGAGAGAGAGAGACGATATCGAGGAGGGGGGA
GATGAGAGACTGAGCTCGAAAGAGGGAGAGAGCGATGGAGGCGAAGCAAGGC
TTGCTTGCGTATTGGCCTTGCGTGTGCTCAAGCTGTCTCTGTGTAGGTCGGTAG
AGGTAACGCAAGCAGGTAAGGTAAGGTAAGATTAGAGTCGAGGAGAGCTACTG
 
CTAGCTTGGTGACACCTCTTTTGTTTGGATCTTGTGGTCGAGGGGCCAAAAGTG
CCTTTTTTTTCTTTTAGGCGTGTCGAAGCGGGGTCCTTGGTTCGTCACAGCCA
GGCAGGAGAGGCACCTAGCCATACAGATTGGTCGTACAGTTTGTTCGCCGAATT
GTCAGGATGTTTAAAAAATGAAGAGCAATCTAAAAAGAGGCTTCAATATATCACA
CCAAACAGCGCAGACAGGTTGTATCTGCCAACCTCGTGCCTGACAGTCATGGAA
CTGTCCGCGGTGATGCCATCCAGCGCAAAGAATAGCCGCTGGTTTAAGGCTCG
GGCAACTTCTATGGCTCTGCTCAGGCGGGGGGAACCTGGCGCCTGTTAGGT
GCCCACCTCAGCGAAAGGCGCTGCGGTTATTAGTGCTGGAGGCGTGTCAAGGC
CGCTGTGGAGCAGCTCAACTTACAGGGGTTGTCCGCTATACTAGTAGAAGGTGA
AGAGCGAAGCTCTAGCGCGGTGTGACGGCGGGTCGTGATCATGCATCTGATGG
GCATCTTGGAAACTGGGAGTTTGCAAGAGAATTTTCCATTGACGAAGCGGTGAA
TGCTCGGGCGTGACGTCTAATTACGTATTCGGAGGTGTCAGACAGATGTTTCTG
GCTCAAGGTTCGTGAGATACGTAGCCTGACGCGGTGATGGGCGGTTAAGCGAT
GGATTTGCGACTCTTGCTGCTACTACTCTGTACATGTAATAACATGCAAATAGTA
CCTAGGTAATGTGAGCAGAGAGCACAGACGGACAGAGATGGATATAGCCTCAA
TTGCTGAGCCATCATAGCGCAGCTACTGCCTATTGTCTACCTATTGTGTCTCTTT
GTAGCCACCTCTTGTTAATGCCTTGCCTTGCCTAGTTTCTCTCCTACCAGACGCC
AACTGAAGTGTTTCCTTTTTCATATCCTCTCCAACAACCAAACTTCAACCTCCCA
CAGCACCCTCCAACCCTCTCTTCAACACAGCCAAACCCATTGAAACAATCACAC
AAAGAGAGCCAGAAAAGGCGGGGTTCCTATAGTCCCATCACCTTCACTCACTCC
CCATCCCTCCCTCCATCACATCACATAACGATACATAACAAGACACAGCCCGGG
CTCTTGTCTGTTAGATATACTACTACTGTTACTGCTCTACGGAGCTCAACCAACA
AAGAATCTTGCCTCACCATCGTACCAATGCCCTCCCAAATTAGGTCGCAAATAG
AGGATAAACACCCAAGGGAAATCAAGTGTACACACTCTCCGTCCAAGAATTCAC
CTGCAGCCCCCGTTCCGCCATCTCCGCGTCCTTCAAAAAGTCCATTGGCATCCA
CACCAGGTGCCCCTTGATCTGCGCGAGCTTCTTCTTCGCCTCGTCCGGCGGCA
TGAACTGGTCGAATATGTGACCCGGCTTGACGCCCCTAGGCGGCAGATAGCGG
TCGTAGTCTTCAAACGTTTTGACTAGTCTCCTGTTAGCTTTCGCTACCTAGTCAC
AAGCATCTCGCTGGCTTCAAGCCCAACTTACTGTGATCGTCTGGATCCGCGTGG
AAGAGGTGCCGGAACAGCTCCGTGTTCTTGTCGGCTCGACTCGTCCACATCTC
CCACAGCTCCTCGCCCAGCGGATCCTCCACGAGCTTCCAGCTCTCGTCATTGTC
GTAGACATCGTTCTTAGGCTCAACACTCGGCGGCTGAGCGTTGATGTCGCCCTC
GGCATCATTGTCCTGCGGCGGCAACAGACCGAGATGCTCCCGCCAGAGGAATC
GCCGCAGAGTCATTGCATGGTAGCCCGCCTCGAACGGCTTGCCGTCCATCGTC
 
GTCTGAACCAAGTTGGTGTCCTCCATGACGATGCTCAACTCGCTGTCGTGGCTG
CCCTGCTGGCTGCGGTCGTTGAGGTTGCTCGAGCCGCAAATCACCACTCGGTC
GTCAGCGATGAGTACCTTGGCGTGCACGTAGAGCTCCTCTTGGATCCAGTTCTT
GATCTCAGAATCGGGATCCGTCCACGGCTCGTCCTCCAGAGCACCCTGGCCAG
CCATGGCGTGGTGCGCAACCGAGGGAGAGGTCTGAACCTCTTCTTTAGGCTTG
GCCTTTTCAAAAGCTTCCTTTGCTTGCACAGCCTCGTTGCGCGCCTTTTCCTGCT
CGTTTGCCTTGCCCATGTGGAGGTCTCTCTCCTTTTCTATCGGGTCAGCCGTCC
CGTGGATACCCTCGCCCATAATGTCCTCGGCCAGAACACGCTGAACTTGCTGGT
ACCCGATGCCCGTCTTCTTCTCGGCTTCAGCAATAGCCGGCGTCTTGTTCAGGC
GGTCGTACGATCTCAAGTTAAAGAAGATGTGTTTGGTGGGGTCGACTCCTT
GAGCCTTGACTCGCTCAAAGATTGAATGCTCGCCGCGGCATATTGACTTGTACT
GGTAATCCATGATGGCCCTGGTGCCGGAGGCGGCCTTGTCTCGAAGGTCGCCT
GCGAATCCTGGAACAGCAGGGATGATGACAATGACTCTGAACTTGCGGCCTTC
CTTCCCGGCCCGGACCACGGCCTCGACGATTGCTTCGCCGATGGTGTTGTGTA
TCGGTGACTGGTGTTCGCCTGTGGCGGTGATGAAGAACTGGTTCTCGATATAGA
CGTAGTGCTGCGCTTTGGAAATGACCTCGGAGTAGGCGTTTAAACGACTTTTA
ATTAACTGGCCTCATGGGCCTTCCGCTCACTGCCCGCTTTCCAGTCGGGAAACC
TGTCGTGCCAGCTGCATTAACATGGTCATAGCTGTTTCCTTGCGTATTGGGCGC
TCTCCGCTTCCTCGCTCACTGACTCGCTGCGCTCGGTCGTTCGGGTAAAGCCT
GGGGTGCCTAATGAGCAAAAGGCCAGCAAAAGGCCAGGAACCGTAAAAAGGCC
GCGTTGCTGGCGTTTTTCCATAGGCTCCGCCCCCCTGACGAGCATCACAAAAAT
CGACGCTCAAGTCAGAGGTGGCGAAACCCGACAGGACTATAAAGATACCAGGC
GTTTCCCCCTGGAAGCTCCCTCGTGCGCTCTCCTGTTCCGACCCTGCCGCTTAC
CGGATACCTGTCCGCCTTTCTCCCTTCGGGAAGCGTGGCGCTTTCTCATAGCTC
ACGCTGTAGGTATCTCAGTTCGGTGTAGGTCGTTCGCTCCAAGCTGGGCTGTGT
GCACGAACCCCCCGTTCAGCCCGACCGCTGCGCCTTATCCGGTAACTATCGTC
TTGAGTCCAACCCGGTAAGACACGACTTATCGCCACTGGCAGCAGCCACTGGT
AACAGGATTAGCAGAGCGAGGTATGTAGGCGGTGCTACAGAGTTCTTGAAGTG
GTGGCCTAACTACGGCTACACTAGAAGAACAGTATTTGGTATCTGCGCTCTGCT
GAAGCCAGTTACCTTCGGAAAAAGAGTTGGTAGCTCTTGATCCGGCAAACAAAC
CACCGCTGGTAGCGGTGGTTTTTTTGTTTGCAAGCAGCAGATTACGCGCAGAAA
AAAAGGATCTCAAGAAGATCCTTTGATCTTTTCTACGGGGTCTGACGCTCAGTG
GAACGAAAACTCACGTTAAGGGATTTTGGTCATGAGATTATCAAAAAGGATCTTC
ACCTAGATCCTTTTAAATTAAAAATGAAGTTTTAAATCAATCTAAAGTATATATGA
 
GTAAACTTGGTCTGACAGTTACCAATGCTTAATCAGTGAGGCACCTATCTCAGC
GATCTGTCTATTTCGTTCATCCATAGTTGCCTGACTCCCCGTCGTGTAGATAACT
ACGATACGGGAGGGCTTACCATCTGGCCCCAGTGCTGCAATGATACCGCGAGA
ACCACGCTCACCGGCTCCAGATTTATCAGCAATAAACCAGCCAGCCGGAAGGG
CCGAGCGCAGAAGTGGTCCTGCAACTTTATCCGCCTCCATCCAGTCTATTAATT
GTTGCCGGGAAGCTAGAGTAAGTAGTTCGCCAGTTAATAGTTTGCGCAACGTTG
TTGCCATTGCTACAGGCATCGTGGTGTCACGCTCGTCGTTTGGTATGGCTTCAT
TCAGCTCCGGTTCCCAACGATCAAGGCGAGTTACATGATCCCCCATGTTGTGCA
AAAAAGCGGTTAGCTCCTTCGGTCCTCCGATCGTTGTCAGAAGTAAGTTGGCCG
CAGTGTTATCACTCATGGTTATGGCAGCACTGCATAATTCTCTTACTGTCATGCC
ATCCGTAAGATGCTTTTCTGTGACTGGTGAGTACTCAACCAAGTCATTCTGAGAA
TAGTGTATGCGGCGACCGAGTTGCTCTTGCCCGGCGTCAATACGGGATAATAC
CGCGCCACATAGCAGAACTTTAAAAGTGCTCATCATTGGAAAACGTTCTTCGGG
GCGAAAACTCTCAAGGATCTTACCGCTGTTGAGATCCAGTTCGATGTAACCCAC
TCGTGCACCCAACTGATCTTCAGCATCTTTTACTTTCACCAGCGTTTCTGGGTGA
GCAAAAACAGGAAGGCAAAATGCCGCAAAAAAGGGAATAAGGGCGACACGGAA
ATGTTGAATACTCATACTCTTCCTTTTTCAATATTATTGAAGCATTTATCAGGGTT
ATTGTCTCATGAGCGGATACATATTTGAATGTATTTAGAAAAATAAACAAATAGG
GGTTCCGCGCACATTTCCCCGAAAAGTGCCAC
SEQ ID NO: 3
   Hygromycin B resistance marker 
GTTAACAAGACACAGCCCTATAACTTCGTATAATGTATGCTATACGAAGTTATAT
AACGGTGAGACTAGCGGCCGGTCCCCTTATCCAGCTGTTCCACGTTGGCCTG
CCCCTCAGTTAGCGCTCAACTCAATGCCCCTCACTGGCGAGGCGAGGCAAGG
ATGGAGGGGCAGCATCGCCTGAGTTGGAGCAAAGCGGCCCGGCCGCCATGGG
AGCAGCGAACCAACGGAGGGATGCCGTGCTTTGTCGTGGCTGCTGTGGCCAAT
CCGGGCCCTTGGTTGGCTCACAGAGCGTTGCTGTGAGACCATGAGCTATTATTG
CTAGGTACAGTATAGAGAGAGGAGAGAGAGAGAGAGAGAGAGAGGGGAAAA
AAGGTGAGGTTGAAGTGAGAAAAAAAAAAAAAAAAAAAAATCCAACCACTGACG
GCTGCCGGCTCTGCCACCCCCCTCCCTCCACCCCAGACCACCTGCACACTCAG
CGCGCAGCATCACCTAATCTTGGCTCGCCTTCCCGCAGCTCAGGTTGTTTTTT
TTTCTCTCTCCCTCGTCGAAGCCGCCCTTGTTCCCTTATTTATTTCCCTCTCCAT
CCTTGTCTGCCTTTGGTCCATCTGCCCCTTTGTCTGCATCTCTTTTGCACGCATC
 
GCCTTATCGTCGTCTCTTTTTCACTCACGGGAGCTTGACGAAGACCTGACTCG
TGAGCCTCACCTGCTGATTTCTCTCCCCCCTCCCGACCGGCTTGACTTTTGTT
TCTCCTCCAGTACCTTATCGCGAAGCCGGAAGAACCTCTTAACCTCTAGATGAA
AAAGCCTGAACTCACCGCCACGTCTGTCGAGAAGTTCCTGATCGAAAAGTTCGA
CAGCGTCTCCGACCTGATGCAGCTCTCGGAGGGCGAAGAATCTCGTGCTTTCA
GCTTCGATGTAGGAGGGCGTGGATATGTCCTGCGGGTAAATAGCTGCGCCGAT
GGTTTCTACAAAGATCGTTATGTTTATCGGCACTTTGCATCGGCCGCGCTCCCG
ATTCCGGAAGTGCTTGACATTGGGGAATTCAGCGAGAGCCTGACCTATTGCATC
TCCCGCCGTGCACAGGGTGTCACGTTGCAAGACCTGCCTGAAACCGAACTGCC
CGCTGTTCTGCAGCCGGTCGCGGAGGCCATGGATGCGATCGCTGCGGCCGAT
CTCAGCCAGACGAGCGGGTTCGGCCCATTCGGACCGCAAGGAATCGGTCAATA
CACTACATGGCGTGATTTCATATGCGCGATTGCTGATCCCCATGTGTATCACTG
GCAAACTGTGATGGACGACACCGTCAGTGCGTCCGTCGCGCAGGCTCTCGATG
AGCTGATGCTTTGGGCCGAGGACTGCCCCGAAGTCCGGCACCTCGTGCACGC
GGATTTCGGCTCCAACAATGTCCTGACGGACAATGGCCGCATAACAGCGGTCAT
TGACTGGAGCGAGGCGATGTTCGGGGATTCCCAATACGAGGTCGCCAACATCT
TCTTCTGGAGGCCGTGGTTGGCTTGTATGGAGCAGCAGACGCGCTACTTCGAG
CGGAGGCACCCGGAGCTTGCAGGATCGCCGCGGCTCCGGGCGTATATGCTCC
GCATTGGTCTTGACCAACTCTATCAGAGCTTGGTTGACGGCAATTTCGATGATG
CAGCTTGGGCGCAGGGTCGATGCGACGCAATCGTCCGATCCGGAGCCGGGAC
TGTCGGGCGTACACAAATCGCCCGCAGAAGCGCGGCCGTCTGGACCGATGGC
TGTGTAGAAGTACTCGCCGATAGTGGAAACCGACGCCCCAGCACTCGTCCGAG
GGCAAAGGAATAGATGCATGGCTTTCGTGACCGGGCTTCAAACAATGATGTGCG
ATGGTGTGGTTCCCGGTTGGCGGAGTCTTTGTCTACTTTGGTTGTCTGTCGCAG
GTCGGTAGACCGCAAATGAGCAACTGATGGATTGTTGCCAGCGATACTATAATT
CACATGGATGGTCTTTGTCGATCAGTAGCTAGTGAGAGAGAGAACATCTATC
CACAATGTCGAGTGTCTATTAGACATACTCCGAGAATAAAGTCAACTGTGTCTGT
GATCTAAAGATCGATTCGGCAGTCGAGTAGCGTATAACAACTCCGAGTACCAGC
GAAAGCACGTCGTGACAGGAGCAGGGCTTTGCCAACTGCGCAACCTTGCTTGA
ATGAGGATACACGGGGTGCAACATGGCTGTACTGATCCATCGCAACCAAAATTT
CTGTTTATAGATCAAGCTGGTAGATTCCAATTACTCCACCTCTTGCGCTTCTCCA
TGACATGTAAGTGCACGTGGAAACCATACCCAATATAACTTCGTATAATGTATGC
TATACGAAGTTATAGGGCTCTTGTCTGTTAAC
SEQ ID NO: 4
   SEQ1 MHygRfw
   AACAAGACACAGCCCTATAAC
SEQ ID NO: 5
   SEQ1 MHygRrv
   AACAGACAAGAGCCCTATAAC
SEQ ID NO: 6
   SEQ1MKOfw
   GATGGCTGTGTAGAAGTAC
SEQ ID NO: 7
   SEQ1MKOrv
   GAGAGGTTTGACTGGATC
SEQ ID NO: 8
   SEQ1 MSeqfw
   TGACATTCTCCTGGACACCC
SEQ ID NO: 9
   SEQ1 MSeqrv
   GTTGCGTCTTCTCTTGCGTC
SEQ ID NO: 10
   MSeq-01
   AAGCATTGACGACAGAAAGG
SEQ ID NO: 11
   Mutated sequence ID 1

ATGACGCAGTCTCCCATGATCGCCGCGCCGCCCAAGGCCACCAACGAGATCGA
CTGGGTCTCGCCGCTCAAGGCCTACATCCGCGACACCTACGGCGACGACCCCG
AGCGCTATGCCGAAGAGTGCGCCACGCTCAACCGCCTGCGGCGGGACATGCG
CGGCGCCGGCAAGGAGAGCGTCACGGGGAGGGACATGCTCTACCGCTACTAC

GGGCAGCTGGAGCTGCTGGACCTGCGCTTCCCGGTCGATGAGCAGCACATTAA
GATTCCCTTTACATGGTGCGTGACGGAGACACGTTGTGGCCCGCAGCCTTTCTG
TCGTCAATGCTTTGCTTGGAGTTGTGGTAGACGCTGACTTGGGATCTTGTGCTA
GGTTTGACGCATTCACCCACAAACCAACCACGCAGTACTCGCTCGCGTTCGAAA
AGGCCTCTGTCATCTTCAACATCTCCGCCGTCCTTTCCGGCCATGCTGCCATCC
AGAACCGAGAGGATGATTCCGCACTCAAGGTCGCCTACCACTCGTTCCAGGCC
TCGGCCGGCATGTTTACGTACATCAACGAGAACTTCTTGCATGCTCCCTCATTC
GACTTGAGCCGAGAGACCGTCAAGACCTTGATACACATCATGCTCGCCCAGGC
ACAGGAAATCTTTCTGGAGAAGCAGGTCAAGGACCAGAAAAAGGCCGGGTTGC
TGGCCAAGCTGGCGTCGCAGTCTGGGTACCTCTATGGACAGGCTGTGGAGGGT
GTCCAGGAGAATGTCACAAAGGCCATCTTTGAGAAGGTCTGGCTGACAATGGTC
CAGGTAAGCTTGGGTGTCTAATGAGGGGATGGATGTTCGCGTGCTTGAGATGC
TGCAGCAGCGAGATTGCTAATTGGGCCGATTGAAACGCCAGATCAAAGCAAGTC
TTCTCAACTCCATGGCGCAATATTATCAGGCAATGGCAGACGACGAAGCGGGC
CAGCATGGCGTGGCCCTATCTCGACTCCAGGTGGCCGATACCCTCGCCAAGGA
CGCCGACCGATTAGCAAAGAGCTTCCCCAGCACCCTACCGTCCAACGCCAATC
TTGGTGCCGACTGCAGTACGCATCTGCAGGAGATTACAAAACGACAGTGCTCGA
CGGTGCAGGAACGGCTACGAGAGGCCATCAAGGACAATGACTACATCTACCAT
CAAACCGTCCCCGCGGAGGCGACCCTCCCCCAAATCGCCAAGCTCCCGGCCG
CAAAGCCCATTCCCGTATCGGAGCTCTACGCAGGCCAAGACATCCAGCGCATC
ACGGGGCCCGATTTGTTCTCCAAGATTGTGCCCATGGCCGTTACCGAGTCCGC
CAGCTTATATGACGAGGAAAAGGCCAAGCTTGTTAGAGCCGAGACGGAAAAGG
TGGATACGGCAAACGGCGAGATGGCGGCCAGCCTGGACTACCTGCGGCTTCC
GGGGGCGCTGCAGGTGCTCAAGGGCGGGTTCGACCAGGACATTCTTCCCGAC
GAGGATTTCCGGCAATGGTGTGAAGACGTGGCCAACCACGAGAATCCCGTGAG
CATCTTTGACTTTTTGCGGAGCGAGAAGGAGTCGATAGTGTCGACTCTGGACAA
GGCCTCCAAGCAGCTGGACATGGAGGAGAGCGTGTGCGAAAAGATGCGGTCC
AAGTACGAAAACGAATGGAGCCAGCAGCCCAGCGCGCGCCTCACGACGACCTT
GCGGGGAGACATTCGCAACTACCGGGAGGCCCTGGAGGAGGCCAGCAGGAGC
GACGGCCAGCTGGCGGCGAAGCTGCGCCAGAACGAGACGTGGTTCGACGAGA
TGCGGAACGCCGTCGCAAACGGACAGGTGGACCAGCTCTTTTCAAGGGCGGTC
TCCCAGGCCAAGGGGCGAAGCAGCAATGCCGTCAGCCCGTCTGGAAACGAGC
CGAACCTGCTCGATGCAGACTTTGACGAATCTGGGCCTACGGTGGTGGAGCAG
ATTGCAAAGGTCGAAGAGATTCTCAAGAAGCTCAACCTCATCAAGAGAGAGCGG

AATCAGGTCCTCAAGGACCTCAAGGAGAAGGTAAGCTTCTGCTACTGAATCTGC
AGGCTTTATCTCGAAGAGGGCGACATTAACACGGATTGTAGGTCCACAACGACG
ACATCTCTCAGGTCCTCATTCTCAACAAAAAGACGATAGCAAACTATGAGCAGCA
ACTTTTCAAGCAGGAATTGGAAAAGTTTCGGCCGCATCAGAATCGCTTGCTACA
GGCAAACCACAAACAGTCGGCCTTGATGAAGGAGCTCACGGCGACGTTCAACA
CTCTGCTGCAGGACAAGCGCGTGCGCGGAGCAGAGCAAGTACGAATCGATC
CAGCGGCAGAGGCTATCGGCGATTGGCAAGTACAAGCGCGCCTATCAGGAGTT
CTTGGACCTAGAGGCGGGCTTGCAGAGCGCCAAGAACTGGTACTCGGAGATGA
GGGAGACGGTGGAGAGCCTCGAGAAGAACGTGGAGACTTTTGTAAACAACCGG
AGGTCGGAGGGCGCCCAGCTGCTCAACCAGATCGAACAGGAGCGCGTCCA
ACAAGAGCCAGCAGGCAGAGCTGGAGAGGGAGCGGCTGCGAGGCCTCATGGA
GCGCATGTCGATGGAGCCCGCGCAGCCGGCACCGCCCGCCAGACCACCGTCG
GGAAGACCAACGCCAGCGCCCCTGATGCAGCAGCAGAACCAGGCCTCTCGATA
CGGTCAGGGCGGCAGCAGCAGCAGCAGCAACAACAACAACAACGGCTATCAG
GGGCAGTTTCAGATGCCTACATCGCCGCCGCCCAACCAGCAGAGCTTTACCGG
ATACGCCAGCCCTCCTCCGCAAAGCACCTTTTCGCAACCCGTCTACAATCCGAG
CACGTACGGTAGGAACCCCGGGCCGACATCGCCGCCTCCAAACCAGACATCTT
TTAGCATGAATGTCATGAGAGGCCCCCAATCGCCGCCACCCACGCAGACATCG
TTCGGACAGCACCAGCCATATACCATGTACGGGGCATTACCCCAGCAGCAGCA
ACAGCAGCAAACGCAGCAGCAGCAACAACAGCACGCACCTGGAGGATATGTGC
CGCCCGGCTTCGTGCCTCCTCCGCCTCCTCCAGGTCCTCCTCCGCTGGGGCCT
CAGCAGACGATTCATTTTGGGCAGCAGGACTTTGATCCTGCGGTTAGCCACCCG
TCAAGCGCGCAGCCTCGGTCAGCCCAACCGCAGCAGGCGCACGATCCTTGGG
CCGGGTTGAATGCGTGGAAATGA

## Claims

1. Process for production of a whole broth enzyme composition, comprising the following steps:
(a) providing a fermentation medium, originating from hydrolysis of lignocellulosic biomass, with a glucose content of from 5 to 450 g/L, a xylose content of from 2 to 300 g/L, a density of from 1 to 2 kg/L and a dry matter content of from 10 to 75 wt.-%;
(b) addition of at least one filamentous fungus cell wherein the A at position 149 of SEQ ID NO:1 has been changed to a G;
(c) mixing of the fermentation medium and the at least one filamentous fungus cell for a time period of from 1 minute to 10 days at a temperature of from 20 to 35 °C;
(d) obtaining a whole broth enzyme composition.

2. Process according to claim 1, wherein the pH of the fermentation medium according to step (a) has been adjusted to a pH selected from pH 2.0 to 6.0.

3. Process according to any of the foregoing claims, wherein the ratio from glucose to xylose is from 1.0 to 3.5.

4. Process according to any of the foregoing claims, further comprising the step (ai) concentration of the fermentation medium by evaporation, membrane filtration or thin layer evaporation to decrease the weight of the fermentation medium by factor 2 to 6.

5. Process according to any of the foregoing claims, further comprising step (aii) sterilization of the fermentation medium according to step (a) or the concentrated fermentation medium according to step (ai).

6. Process according to any of the foregoing claims, wherein the fermentation medium according to step (a) has a furfural content of less than 0.5 g/L.

7. Process according to any of the foregoing claims, wherein the fermentation medium according to step (a) has a hydroxymethyl furfural (HMF) content of less than 0.5 g/L.

8. Process according to any of the foregoing claims further comprising the step
(e) solid-liquid separation of the fermented medium according to step (c) to obtain a solid fraction and a liquid fraction.

9. Process according to any of the foregoing claims wherein from 0.05 to 5 wt.-% nitrogen are added during step (a) and/or (b) of the process.

10. Process according to any of the foregoing claims wherein from 0.5 to 350 mg/L FeSO₄, MnSO₄, MgSO₄ and/or ZnSO₄ are added during step (a) and/or (b) of the process.

11. Process according to any of the foregoing claims, wherein the filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.*

12. Process according to any of the foregoing claims, wherein the filamentous fungus cell comprises at least one heterologous beta-glucosidase enzyme.

13. Filamentous fungus cell wherein the A at position 149 of SEQ ID NO:1 has been changed to a G.

14. Filamentous fungus cell according to claim 13, wherein the at least one filamentous fungus cell is a genetically modified filamentous fungus cell with the ability to express at least one heterologous hydrolase enzyme, at least one heterologous pectinase enzyme, at least one heterologous oxidative enzyme and/or at least one heterologous accessory protein.

15. Filamentous fungus cell according to any of claims 13 to 14, wherein the at least one filamentous fungus cell is a genetically modified filamentous fungus cell comprising at least one heterologous beta glucosidase enzyme encoding sequence, at least one heterologous beta-xylosidase enzyme encoding sequence, at least one heterologous xylanase enzyme encoding sequence, at least one heterologous pectinase enzyme encoding sequence, at least one heterologous lytic polysaccharide monooxygenase enzyme encoding sequence, at least one heterologous oxidative enzyme encoding sequence and/or at least one heterologous accessory protein encoding sequence.

16. Whole broth enzyme composition produced according to a process as defined in any of claims 1 to 12.

17. Use of a filamentous fungus cell as defined in any of claims 13 to 15 for the production of whole broth enzyme composition.

18. Use of a whole broth enzyme composition according to claim 17 for the hydrolyzation of lignocellulosic biomass.


SEQUENCE LISTING

<110> Clariant Produkte (Deutschland) GmbH

<120> PROCESS FOR THE PRODUCTION OF A FILAMENTOUS FUNGUS WHOLE BROTH ENZYME
COMPOSITION WITH LOW BIOMASS FORMATION AND HIGH PROTEIN YIELD

<130> 2020DE626-EP-EPA

<160> 11

<170> BiSSAP 1.3.6

<210> 1
<211> 3259
<212> DNA
<213> Trichoderma reesei


<220>
<223> native gene

<400> 1
atgacgcagt ctcccatgat cgccgcgccg cccaaggcca ccaacgagat cgactgggtc      60

tcgccgctca aggcctacat ccgcgacacc tacggcgacg accccgagcg ctatgccgaa     120

gagtgcgcca cgctcaaccg cctgcggcag gacatgcgcg gcgccggcaa ggagagcgtc     180

acggggaggg acatgctcta ccgctactac gggcagctgg agctgctgga cctgcgcttc     240

ccggtcgatg agcagcacat taagattccc tttacatggt gcgtgacgga gacacgttgt     300

ggcccgcagc ctttctgtcg tcaatgcttt gcttggagtt gtggtagacg ctgacttggg     360

atcttgtgct aggtttgacg cattcaccca caaaccaacc acgcagtact cgctcgcgtt     420

cgaaaaggcc tctgtcatct tcaacatctc cgccgtcctt tccggccatg ctgccatcca     480

gaaccgagag gatgattccg cactcaaggt cgcctaccac tcgttccagg cctcggccgg     540

catgtttacg tacatcaacg agaacttctt gcatgctccc tcattcgact tgagccgaga     600

gaccgtcaag accttgatac acatcatgct cgcccaggca caggaaatct ttctggagaa     660

gcaggtcaag gaccagaaaa aggccgggtt gctggccaag ctggcgtcgc agtctgggta     720

cctctatgga caggctgtgg agggtgtcca ggagaatgtc acaaaggcca tctttgagaa     780

ggtctggctg acaatggtcc aggtaagctt gggtgtctaa tgaggggatg gatgttcgcg     840

tgcttgagat gctgcagcag cgagattgct aattgggccg attgaaacgc cagatcaaag     900

caagtcttct caactccatg gcgcaatatt atcaggcaat ggcagacgac gaagcgggcc     960

agcatggcgt ggccctatct cgactccagg tggccgatac cctcgccaag gacgccgacc    1020

gattagcaaa gagcttcccc agcaccctac cgtccaacgc caatcttggt gccgactgca    1080

gtacgcatct gcaggagatt acaaaacgac agtgctcgac ggtgcaggaa cggctacgag    1140

aggccatcaa ggacaatgac tacatctacc atcaaaccgt ccccgcggag gcgaccctcc    1200

cccaaatcgc caagctcccg gccgcaaagc ccattcccgt atcggagctc tacgcaggcc 1260

aagacatcca gcgcatcacg gggcccgatt tgttctccaa gattgtgccc atggccgtta 1320

ccgagtccgc cagcttatat gacgaggaaa aggccaagct tgttagagcc gagacggaaa 1380

aggtggatac ggcaaacggc gagatggcgg ccagcctgga ctacctgcgg cttccggggg 1440

cgctgcaggt gctcaagggc gggttcgacc aggacattct tcccgacgag gatttccggc 1500

aatggtgtga agacgtggcc aaccacgaga atcccgtgag catctttgac tttttgcgga 1560

gcgagaagga gtcgatagtg tcgactctgg acaaggcctc caagcagctg gacatggagg 1620

agagcgtgtg cgaaaagatg cggtccaagt acgaaaacga atggagccag cagcccagcg 1680

cgcgcctcac gacgaccttg cggggagaca ttcgcaacta ccgggaggcc ctggaggagg 1740

ccagcaggag cgacggccag ctggcggcga agctgcgcca gaacgagacg tggttcgacg 1800

agatgcggaa cgccgtcgca aacggacagg tggaccagct cttttcaagg gcggtctccc 1860

aggccaaggg gcgaagcagc aatgccgtca gcccgtctgg aaacgagccg aacctgctcg 1920

atgcagactt tgacgaatct gggcctacgg tggtggagca gattgcaaag gtcgaagaga 1980

ttctcaagaa gctcaacctc atcaagagag agcggaatca ggtcctcaag gacctcaagg 2040

agaaggtaag cttctgctac tgaatctgca ggctttatct cgaagagggc gacattaaca 2100

cggattgtag gtccacaacg acgacatctc tcaggtcctc attctcaaca aaaagacgat 2160

agcaaactat gagcagcaac ttttcaagca ggaattggaa aagtttcggc cgcatcagaa 2220

tcgcttgcta caggcaaacc acaaacagtc ggccttgatg aaggagctca cggcgacgtt 2280

caacactctg ctgcaggaca agcgcgtgcg cgcggagcag agcaagtacg aatcgatcca 2340

gcggcagagg ctatcggcga ttggcaagta caagcgcgcc tatcaggagt tcttggacct 2400

agaggcgggc ttgcagagcg ccaagaactg gtactcggag atgagggaga cggtggagag 2460

cctcgagaag aacgtggaga cttttgtaaa caaccggagg tcggaggcgg cccagctgct 2520

caaccagatc gaacaggagc gcgcgtccaa caagagccag caggcagagc tggagaggga 2580

gcggctgcga ggcctcatgg agcgcatgtc gatggagccc gcgcagccgg caccgcccgc 2640

cagaccaccg tcgggaagac caacgccagc gcccctgatg cagcagcaga accaggcctc 2700

tcgatacggt cagggcggca gcagcagcag cagcaacaac aacaacaacg gctatcaggg 2760

gcagtttcag atgcctacat cgccgccgcc caaccagcag agctttaccg gatacgccag 2820

ccctcctccg caaagcacct tttcgcaacc cgtctacaat ccgagcacgt acggtaggaa 2880

ccccgggccg acatcgccgc ctccaaacca gacatctttt agcatgaatg tcatgagagg 2940

cccccaatcg ccgccaccca cgcagacatc gttcggacag caccagccat ataccatgta 3000

cggggcatta ccccagcagc agcaacagca gcaaacgcag cagcagcaac aacagcacgc 3060

acctggagga tatgtgccgc ccggcttcgt gcctcctccg cctcctccag gtcctcctcc 3120

gctggggcct cagcagacga ttcattttgg gcagcaggac tttgatcctg cggttagcca 3180

cccgtcaagc gcgcagcctc ggtcagccca accgcagcag gcgcacgatc cttgggccgg 3240

gttgaatgcg tggaaatga 3259

<210> 2
<211> 5937
<212> DNA
<213> Artificial Sequence

<220>
<223> pSEQ1M

<400> 2
ctaaattgta agcgttaata ttttgttaaa attcgcgtta aatttttgtt aaatcagctc 60

attttttaac caataggccg aaatcggcaa aatcccttat aaatcaaaag aatagaccga 120

gatagggttg agtggccgct acagggcgct cccattcgcc attcaggctg cgcaactgtt 180

gggaagggcg tttcggtgcg ggcctcttcg ctattacgcc agctggcgaa agggggatgt 240

gctgcaaggc gattaagttg ggtaacgcca gggttttccc agtcacgacg ttgtaaaacg 300

acggccagtg agcgcgacgt aatacgactc actatagggc gaattggcgg aaggccgtca 360

aggcctaggc gcgccagact gtttaaacct agcacaagat cccaagtcag cgtctaccac 420

aactccaagc aaagcattga cgacagaaag gctgcgggcc acaacgtgtc tccgtcacgc 480

accatgtaaa gggaatctta atgtgctgct catcgaccgg gaagcgcagg tccagcagct 540

ccagctgccc gtagtagcgg tagagcatgt ccctccccgt gacgctctcc ttgccggcgc 600

cgcgcatgtc ccgccgcagg cggttgagcg tggcgcactc ttcggcatag cgctcggggt 660

cgtcgccgta ggtgtcgcgg atgtaggcct tgagcggcga gacccagtcg atctcgttgg 720

tggccttggg cggcgcggcg atcatgggag actgcgtcat cctttttgtg cgactttggg 780

gccgcgcggg tatggcggac ccggggatct tggaggaggg ggcgggttag ggtggtcctg 840

ttgttgttca gacgcaagag aagacgcaac gacttcaatg gccacggtcg agacggccgg 900

cagtgtgtcg catgcgcgtc gcctgcgcgg agggacgggg gggaacgctc ggcgggtgtg 960

cggtgcgatg gcaaagctgt gcgaggcggg agctggcggc ttgatgagag agtgcggagg 1020

atgggggggc cccgtttgct gggatgatcg agtctgatcg atgaagagac agagggagag 1080

agagagagag acgatatcga ggaggggggg agatgagaga ctgagctcga aagagggaga 1140

gagcgatgga ggcgaagcaa ggcttgcttg cgtattggcc ttgcgtgtgc tcaagctgtc 1200

tctgtgtagg tcggtagagg taacgcaagc aggtaaggta aggtaagatt agagtcgagg 1260

agagctactg ctagcttggt gacacctctt ttgtttggat cttgtggtcg aggggccaaa 1320

agtgcctttt tttttctttt aggcgtgtcg aagcggggtc cttggttcgt cacagccagg 1380

caggagaggc acctagccat acagattggt cgtacagttt gttcgccgaa ttgtcaggat 1440

gtttaaaaaa tgaagagcaa tctaaaaaga ggcttcaata tatcacacca aacagcgcag 1500

acaggttgta tctgccaacc tcgtgcctga cagtcatgga actgtccgcg gtgatgccat 1560

ccagcgcaaa gaatagccgc tggtttaagg ctcgggcaac ttctatgggc tctgctcagg 1620

cgggggggaa cctggcgcct gttaggtgcc cacctcagcg aaaggcgctg cggttattag 1680

tgctggaggc gtgtcaaggc cgctgtggag cagctcaact tacaggggtt gtccgctata 1740

ctagtagaag gtgaagagcg aagctctagc gcggtgtgac ggcgggtcgt gatcatgcat 1800

ctgatgggca tcttggaaac tgggagtttg caagagaatt ttccattgac gaagcggtga 1860

atgctcgggc gtgacgtcta attacgtatt cggaggtgtc agacagatgt ttctggctca 1920

aggttcgtga gatacgtagc ctgacgcggt gatgggcggt taagcgatgg atttgcgact 1980

cttgctgcta ctactctgta catgtaataa catgcaaata gtacctaggt aatgtgagca 2040

gagagcacag acggacagag atggatatag cctcaattgc tgagccatca tagcgcagct 2100

actgcctatt gtctacctat tgtgtctctt tgtagccacc tcttgttaat gccttgcctt 2160

gcctagtttc tctcctacca gacgccaact gaagtgtttc ctttttcata tcctctccaa 2220

caaccaaact tcaacctccc acagcaccct ccaaccctct cttcaacaca gccaaaccca 2280

ttgaaacaat cacacaaaga gagccagaaa aggcggggtt cctatagtcc catcaccttc 2340

actcactccc catccctccc tccatcacat cacataacga tacataacaa gacacagccc 2400

gggctcttgt ctgttagata tactactact gttactgctc tacggagctc aaccaacaaa 2460

gaatcttgcc tcaccatcgt accaatgccc tcccaaatta ggtcgcaaat agaggataaa 2520

cacccaaggg aaatcaagtg tacacactct ccgtccaaga attcacctgc agcccccgtt 2580

ccgccatctc cgcgtccttc aaaaagtcca ttggcatcca caccaggtgc cccttgatct 2640

gcgcgagctt cttcttcgcc tcgtccggcg gcatgaactg gtcgaatatg tgacccggct 2700

tgacgcccct aggcggcaga tagcggtcgt agtcttcaaa cgttttgact agtctcctgt 2760

tagctttcgc tacctagtca caagcatctc gctggcttca agcccaactt actgtgatcg 2820

tctggatccg cgtggaagag gtgccggaac agctccgtgt tcttgtcggc tcgactcgtc 2880

cacatctccc acagctcctc gcccagcgga tcctccacga gcttccagct ctcgtcattg 2940

tcgtagacat cgttcttagg ctcaacactc ggcggctgag cgttgatgtc gccctcggca 3000

tcattgtcct gcggcggcaa cagaccgaga tgctcccgcc agaggaatcg ccgcagagtc 3060

attgcatggt agcccgcctc gaacggcttg ccgtccatcg tcgtctgaac caagttggtg 3120

tcctccatga cgatgctcaa ctcgctgtcg tggctgccct gctggctgcg gtcgttgagg 3180

ttgctcgagc cgcaaatcac cactcggtcg tcagcgatga gtaccttggc gtgcacgtag 3240

agctcctctt ggatccagtt cttgatctca gaatcgggat ccgtccacgg ctcgtcctcc 3300

agagcaccct ggccagccat ggcgtggtgc gcaaccgagg gagaggtctg aacctcttct 3360

ttaggcttgg ccttttcaaa agcttccttt gcttgcacag cctcgttgcg cgccttttcc 3420

tgctcgtttg ccttgcccat gtggaggtct ctctcctttt ctatcgggtc agccgtcccg 3480

tggataccct cgcccataat gtcctcggcc agaacacgct gaacttgctg gtacccgatg 3540

cccgtcttct tctcggcttc agcaatagcc ggcgtcttgt tcaggcggtc gtacgatctc 3600

aagttaaaga agaagatgtg tttggtgggg tcgactcctt gagccttgac tcgctcaaag 3660

attgaatgct cgccgcggca tattgacttg tactggtaat ccatgatggc cctggtgccg 3720

gaggcggcct tgtctcgaag gtcgcctgcg aatcctggaa cagcagggat gatgacaatg 3780

actctgaact tgcggccttc cttcccggcc cggaccacgg cctcgacgat tgcttcgccg 3840

atggtgttgt gtatcggtga ctggtgttcg cctgtggcgg tgatgaagaa ctggttctcg 3900

atatagacgt agtgctgcgc tttggaaatg acctcggagt aggcgtttaa acgacttttt 3960

aattaactgg cctcatgggc cttccgctca ctgcccgctt tccagtcggg aaacctgtcg 4020

tgccagctgc attaacatgg tcatagctgt ttccttgcgt attgggcgct ctccgcttcc 4080

tcgctcactg actcgctgcg ctcggtcgtt cgggtaaagc ctggggtgcc taatgagcaa 4140

aaggccagca aaaggccagg aaccgtaaaa aggccgcgtt gctggcgttt ttccataggc 4200

tccgcccccc tgacgagcat cacaaaaatc gacgctcaag tcagaggtgg cgaaacccga 4260

caggactata aagataccag gcgtttcccc ctggaagctc cctcgtgcgc tctcctgttc 4320

cgaccctgcc gcttaccgga tacctgtccg cctttctccc ttcgggaagc gtggcgcttt 4380

ctcatagctc acgctgtagg tatctcagtt cggtgtaggt cgttcgctcc aagctgggct 4440

gtgtgcacga accccccgtt cagcccgacc gctgcgcctt atccggtaac tatcgtcttg 4500

agtccaaccc ggtaagacac gacttatcgc cactggcagc agccactggt aacaggatta 4560

gcagagcgag gtatgtaggc ggtgctacag agttcttgaa gtggtggcct aactacggct 4620

acactagaag aacagtattt ggtatctgcg ctctgctgaa gccagttacc ttcggaaaaa 4680

gagttggtag ctcttgatcc ggcaaacaaa ccaccgctgg tagcggtggt ttttttgttt 4740

gcaagcagca gattacgcgc agaaaaaaag gatctcaaga agatcctttg atcttttcta 4800

cggggtctga cgctcagtgg aacgaaaact cacgttaagg gattttggtc atgagattat 4860

caaaaaggat cttcacctag atccttttaa attaaaaatg aagttttaaa tcaatctaaa 4920

gtatatatga gtaaacttgg tctgacagtt accaatgctt aatcagtgag gcacctatct 4980

cagcgatctg tctatttcgt tcatccatag ttgcctgact ccccgtcgtg tagataacta 5040

cgatacggga gggcttacca tctggcccca gtgctgcaat gataccgcga gaaccacgct 5100

caccggctcc agatttatca gcaataaacc agccagccgg aagggccgag cgcagaagtg 5160

gtcctgcaac tttatccgcc tccatccagt ctattaattg ttgccgggaa gctagagtaa 5220

gtagttcgcc agttaatagt ttgcgcaacg ttgttgccat tgctacaggc atcgtggtgt 5280

cacgctcgtc gtttggtatg gcttcattca gctccggttc ccaacgatca aggcgagtta 5340

catgatcccc catgttgtgc aaaaaagcgg ttagctcctt cggtcctccg atcgttgtca 5400

gaagtaagtt ggccgcagtg ttatcactca tggttatggc agcactgcat aattctctta 5460

ctgtcatgcc atccgtaaga tgcttttctg tgactggtga gtactcaacc aagtcattct 5520

gagaatagtg tatgcggcga ccgagttgct cttgcccggc gtcaatacgg gataataccg 5580

cgccacatag cagaacttta aaagtgctca tcattggaaa acgttcttcg gggcgaaaac 5640

tctcaaggat cttaccgctg ttgagatcca gttcgatgta acccactcgt gcacccaact 5700

gatcttcagc atcttttact ttcaccagcg tttctgggtg agcaaaaaca ggaaggcaaa 5760

atgccgcaaa aaagggaata agggcgacac ggaaatgttg aatactcata ctcttccttt 5820

ttcaatatta ttgaagcatt tatcagggtt attgtctcat gagcggatac atatttgaat 5880

gtatttagaa aaataaacaa ataggggttc cgcgcacatt tccccgaaaa gtgccac 5937

<210> 3
<211> 2388
<212> DNA
<213> Artificial Sequence

<220>
<223> Hygromycin B resistance marker

<400> 3
gttaacaaga cacagcccta taacttcgta taatgtatgc tatacgaagt tatataacgg 60

tgagactagc ggccggtccc cttatcccag ctgttccacg ttggcctgcc cctcagttag 120

cgctcaactc aatgcccctc actggcgagg cgagggcaag gatggagggg cagcatcgcc 180

tgagttggag caaagcggcc cggccgccat gggagcagcg aaccaacgga gggatgccgt 240

gctttgtcgt ggctgctgtg gccaatccgg gcccttggtt ggctcacaga gcgttgctgt 300

gagaccatga gctattattg ctaggtacag tatagagaga ggagagagag agagagagag 360

agagagggga aaaaaggtga ggttgaagtg agaaaaaaaa aaaaaaaaaa aaatccaacc 420

actgacggct gccggctctg ccacccccct ccctccaccc cagaccacct gcacactcag 480

cgcgcagcat cacctaatct tggctcgcct tcccgcagct caggttgttt tttttttctc 540

tctccctcgt cgaagccgcc cttgttccct tatttatttc cctctccatc cttgtctgcc 600

tttggtccat ctgccccttt gtctgcatct cttttgcacg catcgcctta tcgtcgtctc 660

ttttttcact cacgggagct tgacgaagac ctgactcgtg agcctcacct gctgatttct 720

ctccccccct cccgaccggc ttgacttttg tttctcctcc agtaccttat cgcgaagccg 780

gaagaacctc ttaacctcta gatgaaaaag cctgaactca ccgccacgtc tgtcgagaag 840

ttcctgatcg aaaagttcga cagcgtctcc gacctgatgc agctctcgga gggcgaagaa 900

tctcgtgctt tcagcttcga tgtaggaggg cgtggatatg tcctgcgggt aaatagctgc 960

gccgatggtt tctacaaaga tcgttatgtt tatcggcact ttgcatcggc cgcgctcccg 1020

attccggaag tgcttgacat tggggaattc agcgagagcc tgacctattg catctcccgc 1080

cgtgcacagg gtgtcacgtt gcaagacctg cctgaaaccg aactgcccgc tgttctgcag 1140

ccggtcgcgg aggccatgga tgcgatcgct gcggccgatc ttagccagac gagcgggttc 1200

ggcccattcg gaccgcaagg aatcggtcaa tacactacat ggcgtgattt catatgcgcg 1260

attgctgatc cccatgtgta tcactggcaa actgtgatgg acgacaccgt cagtgcgtcc 1320

gtcgcgcagg ctctcgatga gctgatgctt tgggccgagg actgccccga agtccggcac 1380

ctcgtgcacg cggatttcgg ctccaacaat gtcctgacgg acaatggccg cataacagcg 1440

gtcattgact ggagcgaggc gatgttcggg gattcccaat acgaggtcgc caacatcttc 1500

ttctggaggc cgtggttggc ttgtatggag cagcagacgc gctacttcga gcggaggcac 1560

ccggagcttg caggatcgcc gcggctccgg gcgtatatgc tccgcattgg tcttgaccaa 1620

ctctatcaga gcttggttga cggcaatttc gatgatgcag cttgggcgca gggtcgatgc 1680

gacgcaatcg tccgatccgg agccgggact gtcgggcgta cacaaatcgc ccgcagaagc 1740

gcggccgtct ggaccgatgg ctgtgtagaa gtactcgccg atagtggaaa ccgacgcccc 1800

agcactcgtc cgagggcaaa ggaatagatg catggctttc gtgaccgggc ttcaaacaat 1860

gatgtgcgat ggtgtggttc ccggttggcg gagtctttgt ctactttggt tgtctgtcgc 1920

aggtcggtag accgcaaatg agcaactgat ggattgttgc cagcgatact ataattcaca 1980

tggatggtct ttgtcgatca gtagctagtg agagagagag aacatctatc cacaatgtcg 2040

agtgtctatt agacatactc cgagaataaa gtcaactgtg tctgtgatct aaagatcgat 2100

tcggcagtcg agtagcgtat aacaactccg agtaccagcg aaagcacgtc gtgacaggag 2160

cagggctttg ccaactgcgc aaccttgctt gaatgaggat acacggggtg caacatggct 2220

gtactgatcc atcgcaacca aaatttctgt ttatagatca agctggtaga ttccaattac 2280

tccacctctt gcgcttctcc atgacatgta agtgcacgtg gaaaccatac ccaatataac 2340

ttcgtataat gtatgctata cgaagttata gggctcttgt ctgttaac 2388

<210> 4
<211> 21
<212> DNA
<213> Artificial Sequence

<220>

<223> SEQ1MHygRfw

<400> 4
aacaagacac agccctataa c                                                21

<210> 5
<211> 21
<212> DNA
<213> Artificial Sequence

<220>
<223> SEQ1MHygRrv

<400> 5
aacagacaag agccctataa c                                                21

<210> 6
<211> 19
<212> DNA
<213> Artificial Sequence

<220>
<223> SEQ1MKOfw

<400> 6
gatggctgtg tagaagtac                                                   19

<210> 7
<211> 18
<212> DNA
<213> Artificial Sequence

<220>
<223> SEQ1MKOrv

<400> 7
gagaggtttg actggatc                                                    18

<210> 8
<211> 20
<212> DNA
<213> Artificial Sequence

<220>
<223> SEQ1MSeqfw

<400> 8
tgacattctc ctggacaccc                                                  20

<210> 9
<211> 20
<212> DNA
<213> Artificial Sequence

<220>
<223> SEQ1MSeqrv

<400> 9
gttgcgtctt ctcttgcgtc                                                    20


<210> 10
<211> 20
<212> DNA
<213> Artificial Sequence


<220>
<223> MSeq-01

<400> 10
aagcattgac gacagaaagg                                                    20


<210> 11
<211> 3259
<212> DNA
<213> Artificial Sequence


<220>
<223> mutated sequence ID 1

<400> 11
atgacgcagt ctcccatgat cgccgcgccg cccaaggcca ccaacgagat cgactgggtc      60

tcgccgctca aggcctacat ccgcgacacc tacggcgacg accccgagcg ctatgccgaa     120

gagtgcgcca cgctcaaccg cctgcggcgg gacatgcgcg gcgccggcaa ggagagcgtc     180

acggggaggg acatgctcta ccgctactac gggcagctgg agctgctgga cctgcgcttc     240

ccggtcgatg agcagcacat taagattccc tttacatggt gcgtgacgga gacacgttgt     300

ggcccgcagc ctttctgtcg tcaatgcttt gcttggagtt gtggtagacg ctgacttggg     360

atcttgtgct aggtttgacg cattcaccca caaaccaacc acgcagtact cgctcgcgtt     420

cgaaaaggcc tctgtcatct tcaacatctc cgccgtcctt tccggccatg ctgccatcca     480

gaaccgagag gatgattccg cactcaaggt cgcctaccac tcgttccagg cctcggccgg     540

catgtttacg tacatcaacg agaacttctt gcatgctccc tcattcgact tgagccgaga     600

gaccgtcaag accttgatac acatcatgct cgcccaggca caggaaatct ttctggagaa     660

gcaggtcaag gaccagaaaa aggccgggtt gctggccaag ctggcgtcgc agtctgggta     720

cctctatgga caggctgtgg agggtgtcca ggagaatgtc acaaaggcca tctttgagaa     780

ggtctggctg acaatggtcc aggtaagctt gggtgtctaa tgaggggatg gatgttcgcg     840

tgcttgagat gctgcagcag cgagattgct aattgggccg attgaaacgc cagatcaaag     900

caagtcttct caactccatg gcgcaatatt atcaggcaat ggcagacgac gaagcgggcc     960

agcatggcgt ggccctatct cgactccagg tggccgatac cctcgccaag gacgccgacc 1020

gattagcaaa gagcttcccc agcaccctac cgtccaacgc caatcttggt gccgactgca 1080

gtacgcatct gcaggagatt acaaaacgac agtgctcgac ggtgcaggaa cggctacgag 1140

aggccatcaa ggacaatgac tacatctacc atcaaaccgt ccccgcggag gcgaccctcc 1200

cccaaatcgc caagctcccg gccgcaaagc ccattcccgt atcggagctc tacgcaggcc 1260

aagacatcca gcgcatcacg gggcccgatt tgttctccaa gattgtgccc atggccgtta 1320

ccgagtccgc cagcttatat gacgaggaaa aggccaagct tgttagagcc gagacggaaa 1380

aggtggatac ggcaaacggc gagatggcgg ccagcctgga ctacctgcgg cttccggggg 1440

cgctgcaggt gctcaagggc gggttcgacc aggacattct tcccgacgag gatttccggc 1500

aatggtgtga agacgtggcc aaccacgaga atcccgtgag catctttgac tttttgcgga 1560

gcgagaagga gtcgatagtg tcgactctgg acaaggcctc caagcagctg gacatggagg 1620

agagcgtgtg cgaaaagatg cggtccaagt acgaaaacga atggagccag cagcccagcg 1680

cgcgcctcac gacgaccttg cggggagaca ttcgcaacta ccgggaggcc ctggaggagg 1740

ccagcaggag cgacggccag ctggcggcga agctgcgcca gaacgagacg tggttcgacg 1800

agatgcggaa cgccgtcgca aacggacagg tggaccagct cttttcaagg gcggtctccc 1860

aggccaaggg gcgaagcagc aatgccgtca gcccgtctgg aaacgagccg aacctgctcg 1920

atgcagactt tgacgaatct gggcctacgg tggtggagca gattgcaaag gtcgaagaga 1980

ttctcaagaa gctcaacctc atcaagagag agcggaatca ggtcctcaag gacctcaagg 2040

agaaggtaag cttctgctac tgaatctgca ggctttatct cgaagagggc gacattaaca 2100

cggattgtag gtccacaacg acgacatctc tcaggtcctc attctcaaca aaaagacgat 2160

agcaaactat gagcagcaac ttttcaagca ggaattggaa aagtttcggc cgcatcagaa 2220

tcgcttgcta caggcaaacc acaaacagtc ggccttgatg aaggagctca cggcgacgtt 2280

caacactctg ctgcaggaca agcgcgtgcg cgcggagcag agcaagtacg aatcgatcca 2340

gcggcagagg ctatcggcga ttggcaagta caagcgcgcc tatcaggagt tcttggacct 2400

agaggcgggc ttgcagagcg ccaagaactg gtactcggag atgagggaga cggtggagag 2460

cctcgagaag aacgtggaga cttttgtaaa caaccggagg tcggagggcg cccagctgct 2520

caaccagatc gaacaggagc gcgcgtccaa caagagccag caggcagagc tggagaggga 2580

gcggctgcga ggcctcatgg agcgcatgtc gatggagccc gcgcagccgg caccgcccgc 2640

cagaccaccg tcgggaagac caacgccagc gcccctgatg cagcagcaga accaggcctc 2700

tcgatacggt cagggcggca gcagcagcag cagcaacaac aacaacaacg gctatcaggg 2760

gcagtttcag atgcctacat cgccgccgcc caaccagcag agctttaccg gatacgccag 2820

ccctcctccg caaagcacct tttcgcaacc cgtctacaat ccgagcacgt acggtaggaa 2880

ccccgggccg acatcgccgc ctccaaacca gacatctttt agcatgaatg tcatgagagg 2940

cccccaatcg ccgccaccca cgcagacatc gttcggacag caccagccat ataccatgta 3000

cggggcatta ccccagcagc agcaacagca gcaaacgcag cagcagcaac aacagcacgc 3060

acctggagga tatgtgccgc ccggcttcgt gcctcctccg cctcctccag gtcctcctcc 3120

gctggggcct cagcagacga ttcattttgg gcagcaggac tttgatcctg cggttagcca 3180

cccgtcaagc gcgcagcctc ggtcagccca accgcagcag gcgcacgatc cttgggccgg 3240

gttgaatgcg tggaaatga 3259